(19) Europäisches Patentamt
European Patent Office
Office européen des brevets

(11) EP 3 936 614 A1

(12) EUROPEAN PATENT APPLICATION
published in accordance with Art. 153(4) EPC

(43) Date of publication:
**12.01.2022 Bulletin 2022/02**

(21) Application number: **20766630.6**

(22) Date of filing: **06.03.2020**

(51) Int Cl.:
***C12N 15/09*** (2006.01) ***C12Q 1/6813*** (2018.01)
***C12Q 1/6886*** (2018.01)

(86) International application number:
**PCT/JP2020/009566**

(87) International publication number:
**WO 2020/179895 (10.09.2020 Gazette 2020/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.03.2019 JP 2019041982**

(71) Applicants:
- **Toray Industries, Inc.**
  **Tokyo, 103-8666 (JP)**
- **National Cancer Center**
  **Tokyo 104-0045 (JP)**

(72) Inventors:
- **YOSHIMOTO, Makiko**
  **Kamakura-shi, Kanagawa 248-8555 (JP)**
- **KOZONO, Satoko**
  **Kamakura-shi, Kanagawa 248-8555 (JP)**
- **OCHIYA, Takahiro**
  **Tokyo 104-0045 (JP)**
- **KATO, Tomoyasu**
  **Tokyo 104-0045 (JP)**
- **YOKOI, Akira**
  **Tokyo 104-0045 (JP)**
- **MATSUZAKI, Juntaro**
  **Tokyo 104-0045 (JP)**

(74) Representative: **Mewburn Ellis LLP**
**Aurora Building**
**Counterslip**
**Bristol BS1 6BX (GB)**

# (54) KIT, DEVICE, AND METHOD FOR DETECTING UTERINE LEIOMYOSARCOMA

(57) This invention provides a kit and device for detection of uterine leiomyosarcoma, and a method for detecting uterine leiomyosarcoma. This invention provides a kit and device for detection of uterine leiomyosarcoma containing a nucleic acid capable of specifically binding to a miRNA in a sample from a subject or a complementary strand thereof and a method for detecting uterine leiomyosarcoma including measuring the miRNA *in vitro*.

EP 3 936 614 A1

SEQUENCE LISTING

<110> Toray Industries, Inc.
National Cancer Center

<120> Kit, device and method for detecting uterine leiomyosarcoma

<130> PH-8295-PCT

<150> JP 2019-041982
<151> 2019-03-07

<160> 43

<170> PatentIn version 3.5

<210> 1
<211> 19
<212> RNA
<213> Homo sapiens

<400> 1
aauggauuuu uggagcagg 19

<210> 2
<211> 18
<212> RNA
<213> Homo sapiens

<400> 2
aggcuggagu gagcggag 18

<210> 3
<211> 16
<212> RNA
<213> Homo sapiens

<400> 3
accgccugcc caguga 16

<210> 4
<211> 22
<212> RNA
<213> Homo sapiens

<400> 4
aaaccguuac cauuacugag uu 22

<210> 5
<211> 21
<212> RNA
<213> Homo sapiens

<400> 5
ucuugaaguc agaacccgca a 21

<210> 6
<211> 24

<212> RNA
<213> Homo sapiens

<400> 6
cugcaggcag aaguggggcu gaca 24

<210> 7
<211> 23
<212> RNA
<213> Homo sapiens

<400> 7
caacggaauc ccaaaagcag cug 23

<210> 8
<211> 73
<212> RNA
<213> Homo sapiens

<400> 8
uguauccuug aauggauuuu uggagcagga guggacaccu gacccaaagg aaaucaaucc 60

auaggcuagc aau 73

<210> 9
<211> 49
<212> RNA
<213> Homo sapiens

<400> 9
gugaggcugg agugagcgga gaucguacca cugcacucca accugguga 49

<210> 10
<211> 57
<212> RNA
<213> Homo sapiens

<400> 10
agaggcaccg ccugcccagu gacaugcguu uaacggccgc gguacccuaa cugugca 57

<210> 11
<211> 72
<212> RNA
<213> Homo sapiens

<400> 11
cuugggaaug gcaaggaaac cguuaccauu acugaguuua guaaugguaa ugguucucuu 60

gcuauaccca ga 72

<210> 12
<211> 79
<212> RNA
<213> Homo sapiens

<400> 12
ccgggacuuu gugguucug accccacuug gaucacgccg acaacacugg ucuugaaguc 60

agaacccgca aaguccugg 79

<210> 13
<211> 85
<212> RNA
<213> Homo sapiens

<400> 13
gggacggggc cugcaggcag aaguggggcu gacagggcag aggguugcgc cccucacca 60

ccccuucugc cugcagcggu gggcu 85

<210> 14
<211> 71
<212> RNA
<213> Homo sapiens

<400> 14
ggggccugca ggcagaagug gggcugacag ggcagagggu ugcgccccu caccacccu 60

ucugccugca g 71

<210> 15
<211> 92
<212> RNA
<213> Homo sapiens

<400> 15
cggcuggaca gcgggcaacg gaaucccaaa agcagcuguu gucuccagag cauuccagcu 60

gcgcuuggau uucgucccu gcucuccugc cu 92

<210> 16
<211> 16
<212> RNA
<213> Homo sapiens

<400> 16
aauggauuuu uggagc 16

<210> 17
<211> 16
<212> RNA
<213> Homo sapiens

<400> 17
auggauuuuu ggagca 16

<210> 18
<211> 16
<212> RNA
<213> Homo sapiens

<400> 18
uggauuuuug gagcag 16

<210>  19
<211>  16
<212>  RNA
<213>  Homo sapiens

<400>  19
ggauuuuugg agcagg    16

<210>  20
<211>  16
<212>  RNA
<213>  Homo sapiens

<400>  20
gcuggaguga gcggag    16

<210>  21
<211>  16
<212>  RNA
<213>  Homo sapiens

<400>  21
aaaccguuac cauuac    16

<210>  22
<211>  16
<212>  RNA
<213>  Homo sapiens

<400>  22
aaccguuacc auuacu    16

<210>  23
<211>  16
<212>  RNA
<213>  Homo sapiens

<400>  23
accguuacca uuacug    16

<210>  24
<211>  16
<212>  RNA
<213>  Homo sapiens

<400>  24
ccguuaccau uacuga    16

<210>  25
<211>  16
<212>  RNA
<213>  Homo sapiens

<400>  25
cguuaccauu acugag    16

<210> 26
<211> 16
<212> RNA
<213> Homo sapiens

<400> 26
guuaccauua cugagu 16

<210> 27
<211> 16
<212> RNA
<213> Homo sapiens

<400> 27
uuaccauuac ugaguu 16

<210> 28
<211> 17
<212> RNA
<213> Homo sapiens

<400> 28
ucuugaaguc agaaccc 17

<210> 29
<211> 16
<212> RNA
<213> Homo sapiens

<400> 29
caggcagaag uggggc 16

<210> 30
<211> 16
<212> RNA
<213> Homo sapiens

<400> 30
caacggaauc ccaaaa 16

<210> 31
<211> 16
<212> RNA
<213> Homo sapiens

<400> 31
aacggaaucc caaaag 16

<210> 32
<211> 16
<212> RNA
<213> Homo sapiens

<400> 32
acggaauccc aaaagc 16

<210> 33
<211> 16
<212> RNA
<213> Homo sapiens

<400> 33
cggaauccca aaagca 16

<210> 34
<211> 16
<212> RNA
<213> Homo sapiens

<400> 34
ggaaucccaa aagcag 16

<210> 35
<211> 16
<212> RNA
<213> Homo sapiens

<400> 35
gaaucccaaa agcagc 16

<210> 36
<211> 16
<212> RNA
<213> Homo sapiens

<400> 36
aaucccaaaa gcagcu 16

<210> 37
<211> 16
<212> RNA
<213> Homo sapiens

<400> 37
aucccaaaag cagcug 16

<210> 38
<211> 22
<212> RNA
<213> Homo sapiens

<400> 38
uugaauggau uuuuggagca gg 22

<210> 39
<211> 24
<212> RNA
<213> Homo sapiens

<400> 39
agaggcaccg ccugcccagu gaca 24

<210> 40
<211> 28
<212> RNA
<213> Homo sapiens

<400> 40
aaaccguuac cauuacugag uuuaguaa 28

<210> 41
<211> 27
<212> RNA
<213> Homo sapiens

<400> 41
gccugcaggc agaaguggggg cugacag 27

<210> 42
<211> 27
<212> RNA
<213> Homo sapiens

<400> 42
ccugcaggca gaaguggggc ugacagg 27

<210> 43
<211> 28
<212> RNA
<213> Homo sapiens

<400> 43
gggcaacgga aucccaaaag cagcuguu 28

## Description

### TECHNICAL FIELD

The present invention relates to a kit or device for detecting uterine leiomyosarcoma comprising a nucleic acid capable of specifically binding to a specific miRNA or a complementary strand thereof, which is used for examining the presence or absence of uterine leiomyosarcoma in a subject, and a method for detecting uterine leiomyosarcoma, comprising measuring the expression level of the miRNA.

### BACKGROUND ART

Uterine sarcoma is a tumor with a poor prognosis among gynecologic tumors, and standard treatment has not been established due to its low frequency of occurrence. Most uterine sarcomas occur in the body of the uterus, with carcinosarcoma accounting for about half and No. 1 of the developing species, and leiomyosarcoma accounting for about 40% and No. 2. Uterus carcinosarcoma is a tumor pathologically similar to endometrium cancer (endometrial cancer) and can be diagnosed by endometrial histology, and standard treatment is currently performed in the same manner as endometrium cancer. Therefore, it can be understood that leiomyosarcoma requires special attention among uterine sarcomas. According to a report by the Ministry of Health, Labor and Welfare of Japan, the prognosis of patients with uterine leiomyosarcoma is extremely poor, with a 50% survival time of 31 months, which is summarized from stages 1 to 4. In addition, the susceptible age of uterine leiomyosarcoma is in the 50s, which is also characterized by being relatively young among tumor-affected persons.

Uterine leiomyosarcoma is not easy to distinguish from a benign tumor, uterine fibroid, even by diagnostic imaging such as MRI. In the case of a benign uterine fibroid, it can be removed by laparoscopic surgery, and the number of operations has increased dramatically in recent years due to the high patients' QOL. Meanwhile, when uterine leiomyosarcoma is suspected, hysterectomy by laparotomy is mentioned as a reliable treatment and diagnostic method, and many patients are referred to large hospitals. Therefore, for uterine leiomyosarcoma, the patients' QOL is not always high not only in view of treatment and diagnosis but also from a social point of view. However, there is currently no accurate preoperative diagnosis of leiomyosarcoma.

Patent Literature 1 indicates the possibility of detection using a specific micro RNA (miRNA) for endometriosis, but endometriosis is a benign disease originating from the endometrium, which is the epithelial tissue of the uterus, and is a disease completely different from uterine smooth muscle tumor originating from uterine smooth muscle.

Non Patent Literature 1 discloses a plurality of miRNAs exhibiting 4-fold or more variation in uterine leiomyosarcoma-derived cell lines and patient-derived uterine leiomyosarcoma tissues as compared with uterine fibroid-derived tissues in Fig. 4 and Fig. 5 but does not disclose a method for discriminating uterine leiomyosarcoma by these miRNAs.

### CITATION LIST

#### Patent Literature

Patent Literature 1: WO 2018/044979

#### Non Patent Literature

Non Patent Literature 1: de Almeida B. et al., Int. J. Mol. Sci. 2018, 19 (1), 52

### SUMMARY OF INVENTION

#### Problem to be Solved by Invention

As described above, there is a report at the research stage that miRNAs with different expression levels are present in uterine leiomyosarcoma as compared with those in uterine fibroids. However, it is unclear whether these miRNAs exert sufficient performance in the actual clinical setting to discriminate uterine smooth muscle tumors. As shown in Comparative Example 1 of the description of the present application, it has been clarified that the microRNA disclosed in Non Patent Document 1 does not have sufficient discrimination performance.

In one embodiment, the present invention is intended to provide a marker capable of detecting uterine leiomyosarcoma. In one embodiment, an object of the present invention is to provide a new tumor marker for uterine leiomyosarcoma capable of preoperative discrimination between uterine leiomyosarcoma and other diseases such as uterine fibroids required in a clinical site and/or having high reliability and high practicality. In one embodiment, the present invention is also intended to provide a non-invasive kit or device for disease diagnosis useful for diagnosis and treatment of uterine leiomyosarcoma with less amount of sample, and a method for determining (or detecting) uterine leiomyosarcoma, using a nucleic acid capable of specifically binding to such a marker.

Means for Solution to Problem

The present inventors have conducted concentrated studies in order to dissolve the problems described above. As a result, they have discovered genes available as uterine leiomyosarcoma detection markers from blood, which can be collected in a low-invasive manner, and they have discovered that uterine leiomyosarcoma can be detected significantly with the use of such genes. This has led to the completion of the present invention.

<Summary of Invention>

Specifically, the present invention includes the following aspects.

(1) A kit for detection of uterine leiomyosarcoma, comprising a nucleic acid capable of specifically binding to at least one polynucleotide selected from the group consisting of the following uterine leiomyosarcoma markers; miR-1246, miR-4430, miR-4485-5p, miR-451a, miR-4635, miR-6511b-5p, and miR-191-5p or a complementary strand of the polynucleotide.
(2) The kit according to (1), wherein the nucleic acid is a polynucleotide selected from the group consisting of the following polynucleotides (a) to (e):

(a) a polynucleotide consisting of a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(b) a polynucleotide comprising a nucleotide sequence shown in any of SEQ ID NOs: 1 to 7;
(c) a polynucleotide consisting of a nucleotide sequence complementary to a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(d) a polynucleotide comprising a nucleotide sequence complementary to a nucleotide sequence shown in any of SEQ ID NOs: 1 to 7 or a nucleotide sequence derived therefrom by the replacement of u with t; and
(e) a polynucleotide hybridizing under stringent conditions to any of the polynucleotides (a) to (d).

(3) A device for detection of uterine leiomyosarcoma, comprising a nucleic acid capable of specifically binding to at least one polynucleotide selected from the group consisting of the following uterine leiomyosarcoma markers; miR-1246, miR-4430, miR-4485-5p, miR-451a, miR-4635, miR-6511b-5p, and miR-191-5p or a complementary strand of the polynucleotide.
(4) The device according to (3), wherein the nucleic acid is a polynucleotide selected from the group consisting of the following polynucleotides (a) to (e):

(a) a polynucleotide consisting of a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(b) a polynucleotide comprising a nucleotide sequence shown in any of SEQ ID NOs: 1 to 7;
(c) a polynucleotide consisting of a nucleotide sequence complementary to a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(d) a polynucleotide comprising a nucleotide sequence complementary to a nucleotide sequence shown in any of SEQ ID NOs: 1 to 7 or a nucleotide sequence derived therefrom by the replacement of u with t; and
(e) a polynucleotide hybridizing under stringent conditions to any of the polynucleotides (a) to (d).

(5) The device according to (3) or (4), which is used for measurement by a hybridization technique.
(6) The device according to (5), wherein the hybridization technique is a nucleic acid array technique.
(7) A method for detecting uterine leiomyosarcoma, comprising: measuring an expression level of at least one polynucleotide selected from the group consisting of the following uterine leiomyosarcoma markers; miR-1246, miR-4430, miR-4485-5p, miR-451a, miR-4635, miR-6511b-5p, and miR-191-5p, in a sample from a subject; and evaluating *in vitro* whether or not the subject has uterine leiomyosarcoma using the measured expression level.
(8) The method according to (7) comprising: plugging the gene expression level of the at least one polynucleotide in the sample from the subject into a discriminant formula capable of discriminating the presence or absence of uterine leiomyosarcoma distinctively, wherein the discriminant formula is created using gene expression levels in samples from subjects known to have uterine leiomyosarcoma and gene expression levels in samples from subjects having no uterine leiomyosarcoma as training samples; and thereby evaluating as to the presence or absence of uterine leiomyosarcoma *in vitro.*

(9) The method according to (7) or (8), comprising: measuring an expression level of the polynucleotide using a nucleic acid capable of specifically binding to the polynucleotide or a complementary strand of the polynucleotide, wherein the nucleic acid is a polynucleotide selected from the group consisting of the following polynucleotides (a) to (e):

(a) a polynucleotide consisting of a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(b) a polynucleotide comprising a nucleotide sequence shown in any of SEQ ID NOs: 1 to 7;
(c) a polynucleotide consisting of a nucleotide sequence complementary to a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(d) a polynucleotide comprising a nucleotide sequence complementary to a nucleotide sequence shown in any of SEQ ID NOs: 1 to 7 or a nucleotide sequence derived therefrom by the replacement of u with t; and
(e) a polynucleotide hybridizing under stringent conditions to any of the polynucleotides (a) to (d).

(10) The method according to any one of (7) to (9), comprising: measuring an expression level of a target gene in the sample from the subject using the kit according to (1) or (2) or the device according to any one of (3) to (6), wherein the kit or the device comprises a nucleic acid capable of specifically binding to the polynucleotide or a complementary strand of the polynucleotide.
(11) The method according to any of (7) to (10), wherein the subject is a human.
(12) The method according to any of (7) to (11), wherein the sample is blood, serum, or plasma.
(13) A uterine leiomyosarcoma detection marker comprising at least one polynucleotide selected from the group consisting of miR-1246, miR-4430, miR-4485-5p, miR-451a, miR-4635, miR-6511b-5p, and miR-191-5p.
(14) The marker according to (13), wherein the polynucleotide is at least one polynucleotide selected from the group consisting of the following polynucleotides (a) and (b):

(a) a polynucleotide consisting of a nucleotide sequence shown in any of SEQ ID NOs: 1 to 7; and
(b) a polynucleotide consisting of a nucleotide sequence that is any of SEQ ID Nos: 1 to 7.

<Definition of Terms>

The terms used herein are defined as described below.
Abbreviations or terms such as nucleotide, polynucleotide, DNA, and RNA abide by "Guidelines for the preparation of specification which contain nucleotide and/or amino acid sequences" (edited by Japan Patent Office) and common use in the art.
The term "polynucleotide" used herein refers to a nucleic acid including any of RNA, DNA, and RNA/DNA (chimera). The DNA includes any of cDNA, genomic DNA, and synthetic DNA. The RNA includes any of total RNA, mRNA, rRNA, miRNA, siRNA, snoRNA, snRNA, non-coding RNA, and synthetic RNA. Here, the "synthetic DNA" and the "synthetic RNA" refer to a DNA and an RNA artificially prepared using, for example, an automatic nucleic acid synthesizer, on the basis of predetermined nucleotide sequences (which may be either a natural or non-natural sequence). The "non-natural sequence" is intended to be used in a broad sense and includes, for example, a sequence comprising substitution, deletion, insertion, and/or addition of one or more nucleotides (i.e., a variant sequence) and a sequence comprising one or more modified nucleotides (i.e., a modified sequence), which are different from the natural sequence. Herein, the term "polynucleotide" is used interchangeably with the term "nucleic acid."
The term "fragment" used herein is a polynucleotide having a nucleotide sequence that consists of a consecutive portion of a polynucleotide and desirably has a length of 15 or more nucleotides, preferably 17 or more nucleotides, and more preferably 19 or more nucleotides.
The term "gene" used herein is intended to include not only RNA and double-stranded DNA but also each single-stranded DNA such as a plus(+) strand (or a sense strand) or a complementary strand (or an antisense strand) constituting the duplex. The gene is not particularly limited by its length.
Thus, the "gene" used herein includes any of double-stranded DNA including human genomic DNA, single-stranded DNA (plus strand), single-stranded DNA having a sequence complementary to the plus strand (complementary strand), cDNA, microRNA (miRNA), their fragments, and human genome, and their transcripts, unless otherwise specified. The "gene" includes not only a "gene" shown in a particular nucleotide sequence (or SEQ ID NO) but also "nucleic acids" encoding RNAs having biological functions equivalent to RNA encoded by the gene, for example, a congener (i.e., a homolog or an ortholog), a variant (e.g., a genetic polymorph), and a derivative. Specific examples of such a "nucleic acid" encoding a congener, a variant, or a derivative can include a "nucleic acid" having a nucleotide sequence hybridizing under stringent conditions described below to a complementary sequence of a nucleotide sequence shown in any of SEQ ID NOs: 1 to 43 or a nucleotide sequence derived therefrom by the replacement of u with t. Regardless of whether or not there is a difference in functional region, the "gene" can comprise, for example, expression control regions, coding regions, exons, or introns. The "gene" may be contained in a cell or may exist alone after being released from a cell. Alternatively, the "gene" may be in a state enclosed in a vesicle referred to as an exosome.

The term "exosome" used herein is a vesicle that is encapsulated by the lipid bilayer and secreted from a cell. The exosome is derived from a multivesicular endosome and may incorporate biomaterials such as "genes" (e.g., RNA or DNA) or proteins when released to an extracellular environment. The exosome is known to be contained in a body fluid such as blood, serum, plasma, or lymph.

The term "transcript" used herein refers to an RNA synthesized from the DNA sequence of a gene as a template. RNA polymerase binds to a site referred to as a promoter located upstream of the gene and adds ribonucleotides complementary to the nucleotide sequence of the DNA to the 3' end to synthesize an RNA. This RNA contains not only the gene itself but also the whole sequence from a transcription initiation site to the end of a poly A sequence, including expression control regions, coding regions, exons, or introns.

Unless otherwise specified, the term "microRNA (miRNA)" used herein is intended to mean a 15- to 25-nucleotide non-coding RNA that is transcribed as an RNA precursor having a hairpin-like structure, cleaved by a dsRNA-cleaving enzyme having RNase III cleavage activity, integrated into a protein complex referred to as RISC, and involved in suppression of mRNA translation. The term "miRNA" used herein includes not only a "miRNA" shown in a particular nucleotide sequence (or SEQ ID NO) but also a "miRNA" comprising a precursor of the "miRNA" (pre-miRNA or pri-miRNA) and having biological functions equivalent to miRNAs encoded thereby, such as a "miRNA" encoding a congener (i.e., a homolog or an ortholog), a variant such as a genetic polymorph, and a derivative. Such a "miRNA" encoding a precursor, a congener, a variant, or a derivative can be specifically identified using "miRBase release 21" (http://www.mirbase.org/), and examples thereof can include a "miRNA" having a nucleotide sequence hybridizing under stringent conditions described below to a complementary sequence of any particular nucleotide sequence shown in any of SEQ ID NOs: 1 to 43. The term "miRNA" used herein may be a gene product of a miR gene. Such a gene product includes a mature miRNA (e.g., a 15- to 25-nucleotide or 19- to 25-nucleotide non-coding RNA involved in suppression of mRNA translation as described above) or a miRNA precursor (e.g., pre-miRNA or pri-miRNA as described above).

The term "probe" used herein includes a polynucleotide that is used for specifically detecting an RNA resulting from the expression of a gene or a polynucleotide derived from the RNA, and/or a polynucleotide complementary thereto.

The term "primer" used herein includes consecutive polynucleotides that specifically recognize and amplify an RNA resulting from the expression of a gene or a polynucleotide derived from the RNA, and/or a polynucleotide complementary thereto.

In this context, the "complementary polynucleotide" (complementary strand or reverse strand) means a polynucleotide in a complementary relationship based on A:T (U) and G:C base pairs with the full-length sequence of a polynucleotide consisting of a nucleotide sequence defined by any of SEQ ID NOs: 1 to 43 or a nucleotide sequence derived therefrom by the replacement of u with t, or a partial sequence thereof (here, this full-length or partial sequence is referred to as a plus strand for the sake of convenience). However, such a complementary strand is not limited to a sequence completely complementary to the nucleotide sequence of the target plus strand and may have a complementary relationship to an extent that permits hybridization under stringent conditions to the target plus strand.

The term "stringent conditions" used herein refers to conditions under which a nucleic acid probe hybridizes to its target sequence to a detectably larger extent (e.g., a measurement value equal to or larger than "(a mean of background measurement values) + (a standard deviation of the background measurement values) $\times$ 2") than that for other sequences. The stringent conditions are dependent on a sequence and differ depending on an environment where hybridization is performed. A target sequence complementary 100% to the nucleic acid probe can be identified by controlling the stringency of hybridization and/or washing conditions. Specific examples of the "stringent conditions" will be mentioned below.

The term "Tm value" used herein means a temperature at which the double-stranded moiety of a polynucleotide is denatured into single strands so that the double strands and the single strands exist at a ratio of 1:1.

The term "variant" used herein means, in the case of a nucleic acid, a natural variant attributed to polymorphism, mutation, or the like; a variant comprising deletion, substitution, addition, or insertion of 1, 2, 3, or more (e.g., 1 to several) nucleotides in a nucleotide sequence shown in any of SEQ ID NOs: 1 to 7, a nucleotide sequence derived therefrom by the replacement of u with t, or a partial sequence thereof; a variant comprising deletion, substitution, addition, or insertion of 1, 2, or more nucleotides in a nucleotide sequence of a precursor RNA (a premature miRNA) of the sequence of any of SEQ ID NOs: 1 to 7, a nucleotide sequence derived therefrom by the replacement of u with t, or a partial sequence thereof; a variant exhibiting percent (%) identity of approximately 90% or higher, approximately 95% or higher, approximately 97% or higher, approximately 98% or higher, or approximately 99% or higher to each of these nucleotide sequences or the partial sequences thereof; or a nucleic acid hybridizing under the stringent conditions defined above to a polynucleotide or an oligonucleotide comprising each of these nucleotide sequences or the partial sequences thereof.

The term "several" used herein means an integer of approximately 10, 9, 8, 7, 6, 5, 4, 3, or 2.

The variant as used herein can be prepared by a well-known technique such as site-directed mutagenesis or mutagenesis using PCR.

The term "percent (%) identity" used herein can be determined with or without an introduced gap, using a protein or gene search system based on BLAST (https://blast.ncbi.nlm.nih.gov/Blast.cgi) or FASTA (http://www.genome.jp/tools/fasta/) (Zheng Zhang et al., 2000, J. Comput. Biol., Vol. 7, pp. 203-214; Altschul, S.F. et al., 1990, Journal of Molecular Biology, Vol. 215, pp. 403-410; and Pearson, W.R. et al., 1988, Proc. Natl. Acad. Sci. U.S.A., Vol. 85, pp. 2444-2448).

The term "derivative" used herein is meant to include unlimitedly a modified nucleic acid, for example, a derivative labeled with a fluorophore or the like, a derivative containing a modified nucleotide (e.g., a nucleotide containing a group such as halogen, alkyl such as methyl, alkoxy such as methoxy, thio, or carboxymethyl, and a nucleotide that has undergone base rearrangement, double bond saturation, deamination, replacement of an oxygen molecule with a sulfur molecule, etc.), PNA (peptide nucleic acid; Nielsen, P.E. et al., 1991, Science, Vol. 254, pp. 1497-500), and LNA (locked nucleic acid; Obika, S et al., 1998, Tetrahedron Lett., Vol. 39, pp. 5401-5404).

As used herein, the "nucleic acid" capable of specifically binding to a polynucleotide selected from the uterine leiomyosarcoma marker miRNAs described above or to a complementary strand of the polynucleotide is a synthesized or prepared nucleic acid, for example, includes a "nucleic acid probe" or a "primer", and is utilized directly or indirectly for detecting the presence or absence of uterine leiomyosarcoma in a subject, for diagnosing the presence or absence or the severity of uterine leiomyosarcoma, the presence or absence or the degree of amelioration of uterine leiomyosarcoma, or the therapeutic sensitivity of uterine leiomyosarcoma, or for screening for a candidate substance useful in prevention, amelioration, or treatment of uterine leiomyosarcoma. The "nucleic acid" includes a nucleotide, an oligonucleotide, and a polynucleotide capable of specifically recognizing and binding to a transcript shown in any of SEQ ID NOs: 1 to 43 or a synthetic cDNA nucleic acid thereof, or a complementary strand thereto *in vivo,* particularly, in a sample such as a body fluid *(e.g.,* blood or urine), in relation to the development of uterine leiomyosarcoma. The nucleotide, the oligonucleotide, and the polynucleotide can be effectively used as probes for detecting the aforementioned gene expressed *in vivo,* in tissues, in cells, or the like on the basis of the properties described above, or as primers for amplifying the aforementioned gene expressed *in vivo.*

The term "detection" used herein is interchangeable with the term "examination," "measurement," or "detection or decision support." As used herein, the term "evaluation" is meant to include diagnosis- or evaluation-support on the basis of examination results or measurement results.

The term "subject" used herein means a mammal such as a primate including a human and a chimpanzee, a pet animal including a dog and a cat, a livestock animal including cattle, a horse, sheep, and a goat, a rodent including a mouse and a rat, and animals raised in a zoo. The subject is preferably a human. Meanwhile, the "healthy subject" also means such a mammal, which is an animal without cancer to be detected. The healthy subject is preferably a human.

As used herein, the "uterine sarcoma" refers to all malignant tumors that occur in the smooth muscle or connective tissue of the uterus, including, endometrial stromal sarcoma and adenosarcoma in addition to leiomyosarcoma targeted by the present invention.

As used herein, the "uterine leiomyosarcoma" is a malignant tumor that develops in uterine smooth muscle.

As used herein, the "uterine fibroids" are benign tumors that develop in the myometrium and are classified into submucosal fibroids, intramuscular fibroids, and subserosal fibroids depending on the location of occurrence.

As used herein, the "smooth muscle tumor of unknown malignancy (STUMP)" refers to a uterine smooth muscle tumor that cannot be determined to be malignant or benign by histopathological diagnosis.

As used herein, the "LDH", also called lactate dehydrogenase, is an enzyme that is always present intracellularly but deviates into the blood when a disease causes damage to an organ or tissue. Therefore, it is used as a disease marker by measuring its abundance in serum. In uterine sarcoma, the amount of LDH in serum may increase abnormally, and it is used as a blood marker for uterine sarcoma.

The term "P" or "P value" used herein refers to a probability at which a more extreme statistic than that actually calculated from data under null hypothesis is observed in a statistical test. Thus, a smaller "P" or "P value" is regarded as being a more significant difference between subjects to be compared.

The term "sensitivity" used herein means a value of (the number of true positives)/(the number of true positives + the number of false negatives). High sensitivity allows uterine leiomyosarcoma to be detected early, which leads to complete resection of cancer regions or a lowered recurrence rate.

The term "specificity" used herein means a value of (the number of true negatives)/(the number of true negatives + the number of false positives). High specificity prevents needless additional testing, patient transfer, and treatment for healthy subjects and uterine fibroid patients misjudged as being uterine leiomyosarcoma patients, which leads to reduction in burden to patients and reduction in medical expenses, such as adaptation of less invasive surgery.

The term "accuracy" used herein means a value of (the number of true positives + the number of true negatives) / (the total number of cases). The accuracy indicates the ratio of samples that are identified correctly to all samples and serves as a primary index for evaluating detection performance.

The term "area under the curve (AUC)" indicates an area under the receiver operating characteristic (ROC) curve, which is obtained by plotting sensitivity of certain results on the y-axis and a false positive fraction or (1-specificity) thereof on the x-axis and lowering the discriminant thresholds as parameters. The AUC value lies between 0.5 and 1, and a value closer to 1 indicates a higher discrimination capacity.

As used herein, the "sample" that is subjected to determination, detection, or diagnosis refers to a tissue and a biological material in which the expression of the gene of the present invention varies as uterine leiomyosarcoma develops, as uterine leiomyosarcoma progresses, or as therapeutic effects on uterine leiomyosarcoma are exerted. Specifically, the sample refers to prostate tissue, renal pelvis, urinary tract, lymph node, organs in the vicinity thereof, organs suspected of metastasis, skin, a body fluid such as blood, urine, saliva, sweat, and tissue exudate, serum or plasma prepared from blood, and others such as feces, hair, or the like. The "sample" further refers to a biological sample extracted therefrom, specifically, a gene such as RNA or miRNA.

The term "hsa-miR-1246 gene" or "hsa-miR-1246" used herein includes the hsa-miR-1246 gene (miRBase Accession No. MIMAT0005898) shown in SEQ ID No: 1, a homolog or an ortholog of a different organism species, and the like. The hsa-miR-1246 gene can be obtained by a method described in Morin RD et al., 2008, GenomeRes, Volume 18, p. 610-621. Further, "hsa-mir-1246" (miRBase Accession No. MI0006381, SEQ ID No: 8) having a hairpin-like structure is known as a precursor of "hsa-miR-1246".

The term "hsa-miR-4430 gene" or "hsa-miR-4430" used herein includes the hsa-miR-4430 gene (miRBase Accession No. MIMAT0018945) shown in SEQ ID No: 2, a homolog or an ortholog of a different organism species, and the like. The hsa-miR-4430 gene can be obtained by a method described in Jima DD et al., 2010, Blood, Volume 116, e118-e127. Further, "hsa-mir-4430" (miRBase Accession No. MI0016769, SEQ ID No: 9) having a hairpin-like structure is known as a precursor of "hsa-miR-4430".

The term "hsa-miR-4485-5p gene" or "hsa-miR-4485-5p" used herein includes the hsa-miR-4485-5p gene (miRBase Accession No. MIMAT0032116) shown in SEQ ID No: 3, a homolog or an ortholog of a different organism species, and the like. The hsa-miR-4485-5p gene can be obtained by a method described in Jima DD et al., 2010, Blood, Volume 116, e118-e127. Further, "hsa-mir-4485" (miRBase Accession No. MI0016846, SEQ ID No: 10) having a hairpin-like structure is known as a precursor of "hsa-miR-4485-5p".

The term "hsa-miR-451a gene" or "hsa-miR-451a" used herein includes the hsa-miR-451a gene (miRBase Accession No. MIMAT0001631) shown in SEQ ID No: 4, a homolog or an ortholog of a different organism species, and the like. The hsa-miR-451a gene can be obtained by a method described in Altuvia Y et al., 2005, Nucleic Acids Res., Volume 33, p. 2697-2706. Further, "hsa-mir-451a (miRBase Accession No. MI0001729, SEQ ID No: 11) having a hairpin-like structure is known as a precursor of "hsa-miR-451a".

The term "hsa-miR-4635 gene" or "hsa-miR-4635" used herein includes the hsa-miR-4635 gene (miRBase Accession No. MIMAT0019692) shown in SEQ ID No: 5, a homolog or an ortholog of a different organism species, and the like. The hsa-miR-4635 gene can be obtained by a method described in Persson H et al., 2011, Cancer Res, Volume 71, p. 78-86. Further, "hsa-mir-4635 (miRBase Accession No. MI0017262, SEQ ID No: 12) having a hairpin-like structure is known as a precursor of "hsa-miR-4635".

The term "hsa-miR-6511b-5p gene" or "hsa-miR-6511b-5p" used herein includes the hsa-miR-6511b-5p5 gene (miRBase Accession No. MIMAT0025847) shown in SEQ ID No: 6, a homolog or an ortholog of a different organism species, and the like. The hsa-miR-6511b-5p gene can be obtained by a method described in Li Y et al., 2012, Gene, Volume 497, p. 330-335. Further, "hsa-miR-6511b-1, miRBase Accession No. MI0022552, SEQ ID No: 13) and "hsa-miR-6511b-2, miRBase Accession No. MI0023431, SEQ ID No: 14) each having a hairpin-like structure is known as precursors of "hsa-miR-6511b-5p".

The term "hsa-miR-191-5p gene" or "hsa-miR-191-5p" used herein includes the hsa-miR-191-5p gene (miRBase Accession No. MIMAT0000440) shown in SEQ ID No: 7, a homolog or an ortholog of a different organism species, and the like. The hsa-miR-191-5p gene can be obtained by a method described in Lagos-Quintana M et al., 2003, RNA, Volume 9, p. 175-179. Further, "hsa-mir-191 (miRBase Accession No. MI0000465, SEQ ID No: 15) having a hairpin-like structure is known as a precursor of "hsa-miR-191-5p".

A mature miRNA may become a variant due to the sequence cleaved shorter or longer by one to several flanking nucleotides or due to substitution of nucleotides, when it is cleaved as the mature miRNA from its RNA precursor having a hairpin-like structure. This variant is referred to as isomiR (Morin RD et al., 2008, Genome Res., Vol. 18, pp. 610-621). The miRBase Release 21 shows the nucleotide sequences shown in any of SEQ ID NOs: 1 to 7 as well as a large number of the nucleotide sequence variants and fragments shown in any of SEQ ID NOs: 16 to 43, referred to as isomiRs. These variants can also be obtained as miRNAs each having a nucleotide sequence shown in any of SEQ ID NOs: 1 to 7. Among the variants of polynucleotides comprising the nucleotide sequence shown in any of SEQ ID NOs: 1 to 7 or a nucleotide sequence derived from any of the nucleotide sequences mentioned above by the replacement of u with t according to the present invention, specific examples of the longest variants registered in the miRBase Release 21 include polynucleotides shown in SEQ ID NOs: 38 to 43. Among the variants of polynucleotides comprising the nucleotide sequence shown in any of SEQ ID NOs: 1 to 7 or a nucleotide sequence derived from any of the nucleotide sequences mentioned above by the replacement of u with t according to the present invention, examples of the shortest variants registered in the miRBase Release 21 include polynucleotides shown in SEQ ID NOs: 16 to37. In addition to these variants and fragments, a large number of isomiR polynucleotides of SEQ ID NOs: 1 to 7 registered in the miRBase are included. Further examples of polynucleotides each comprising a nucleotide sequence shown in any of SEQ ID NOs: 1 to 7 include precursors thereof, which are polynucleotides each shown in any of SEQ ID NOs: 8 to 16.

The names and miRBase Accession Nos. (registration numbers) of the genes shown in SEQ ID NOs: 1 to 43 are shown in Table 1.

As used herein, the expression "capable of specifically binding" refers to a situation in which the nucleic acid probe or primer used in the present invention binds to a specific target nucleic acid and it cannot substantially bind to other nucleic acids.

[Table 1]

| SEQ ID NO: | Name of gene | MIMAT NO. |
|---|---|---|
| 1 | hsa-miR-1246 | MIMAT0005898 |
| 2 | hsa-miR-4430 | MIMAT0018945 |
| 3 | hsa-miR-4485-5p | MIMAT0032116 |
| 4 | hsa-miR-451a | MIMAT0001631 |
| 5 | hsa-miR-4635 | MIMAT0019692 |
| 6 | hsa-miR-6511b-5p | MIMAT0025847 |
| 7 | hsa-miR-191-5p | MIMAT0000440 |
| 8 | hsa-mir-1246 | MI0006381 |
| 9 | hsa-mir-4430 | MI0016769 |
| 10 | hsa-mir-4485 | MI0016846 |
| 11 | hsa-mir-451a | MI0001729 |
| 12 | hsa-mir-4635 | MI0017262 |
| 13 | hsa-mir-6511b-1 | MI0022552 |
| 14 | hsa-mir-6511b-2 | MI0023431 |
| 15 | hsa-mir-191 | MI0000465 |
| 16 | hsa-miR-1246_min1 | |
| 17 | hsa-miR-1246_min2 | |
| 18 | hsa-miR-1246_min3 | |
| 19 | hsa-miR-1246_min4 | |
| 20 | hsa-miR-4430_min | |
| 21 | hsa-miR-451a_min1 | |
| 22 | hsa-miR-451a_min2 | |
| 23 | hsa-miR-451a_min3 | |
| 24 | hsa-miR-451a_min4 | |
| 25 | hsa-miR-451a_min5 | |
| 26 | hsa-miR-451a_min6 | |

(continued)

| SEQ ID NO: | Name of gene | MIMAT NO. |
|---|---|---|
| 27 | hsa-miR-451a_min7 | |
| 28 | hsa-miR-4635_min | |
| 29 | hsa-miR-6511b-5p_min | |
| 30 | hsa-miR-191-5p_min1 | |
| 31 | hsa-miR-191-5p_min2 | |
| 32 | hsa-miR-191-5p_min3 | |
| 33 | hsa-miR-191-5p_min4 | |
| 34 | hsa-miR-191-5p_min5 | |
| 35 | hsa-miR-191-5p_min6 | |
| 36 | hsa-miR-191-5p_min7 | |
| 37 | hsa-miR-191-5p_min8 | |
| 38 | hsa-mir-1246_max | |
| 39 | hsa-mir-4485_max | |
| 40 | hsa-mir-451a_max | |
| 41 | hsa-mir-6511b-5p_max1 | |
| 42 | hsa-mir-6511b-5p_max2 | |
| 43 | hsa-mir-191_max | |

This specification includes the disclosure of Japanese Patent Application No. 2019-041982, which is the basis of the priority of the present application.

Advantageous Effect of Invention

The present invention enables easy detection of uterine leiomyosarcoma with high accuracy. For example, whether or not a subject has uterine leiomyosarcoma can be detected easily with the use of the measured expression level of one or several miRNAs in the blood, serum, and/or plasma that can be collected less invasively from the subject as an indicator.

BRIEF DESCRIPTION OF DRAWINGS

[Figure 1] Fig. 1 shows the relationship between the nucleotide sequence of a precursor hsa-mir-1246 shown in SEQ ID NO: 8 and the nucleotide sequence of hsa-miR-1246 shown in SEQ ID NO: 1 which is generated by the sequence of SEQ ID NO: 8.

[Figure 2] Fig. 2 illustrates clusters by a heat map method, which are obtained by an unsupervised cluster formation method for the expression levels of 7 types of miRNAs in 29 samples derived from tumors in the uterus.

[Figure 3] Fig. 3(A) shows a receiver operating characteristic (ROC) curve when uterine leiomyosarcoma is discriminated from uterus adenosarcoma, uterus endometrial stromal sarcoma (ESS), smooth muscle tumor of unknown malignancy (STUMP), and a uterine fibroid, in the case of using miR-1246 in combination with miR-191-5p (a solid line) and using the amount of LDH detected (dotted line), of 29 samples derived from tumors in the uterus and sensitivity and 1-specificity at the maximal area under the curve (AUC) under the ROC curve. Figure 3(B) shows the discrimination results of each disease.

DESCRIPTION OF EMBODIMENTS

Hereafter, the present invention is further described in detail.

1. Target nucleic acid for uterine leiomyosarcoma

The major target nucleic acids as uterine leiomyosarcoma markers for detecting uterine leiomyosarcoma or the presence and/or absence of uterine leiomyosarcoma cells using the nucleic acid probes or primers for detection of uterine leiomyosarcoma as defined above according to the present invention include at least one miRNA selected from the group consisting of miR-1246, miR-4430, miR-4485-5p, miR-451a, miR-4635, miR-6511b-5p, and miR-191-5p.

Examples of the above miRNAs include any human gene comprising any nucleotide sequence shown in any of SEQ ID NOs: 1 to 7 (*i.e.*, miR-1246, miR-4430, miR-4485-5p, miR-451a, miR-4635, miR-6511b-5p, and miR-191-5p, respectively), a congener thereof, a transcript thereof, and a variant or derivative thereof. Here, the gene, congener, transcript, variant, and derivative are as defined above.

Preferable target nucleic acid is any human gene comprising any nucleotide sequence shown in any of SEQ ID NOs: 1 to 7 or any transcript thereof, and more preferably is the corresponding transcript, namely miRNA and any precursor RNA such as pri-miRNA or pre-miRNA thereof.

The first target gene is the hsa-miR-1246 gene, a congener thereof, a transcript thereof, or a variant or derivative thereof. None of the previously known reports show that change in the expression of the gene or the transcript thereof can serve as a marker for uterine leiomyosarcoma.

The second target gene is the hsa-miR-4430 gene, a congener thereof, a transcript thereof, or a variant or derivative thereof. None of the previously known reports show that change in the expression of the gene or the transcript thereof can serve as a marker for uterine leiomyosarcoma.

The 3rd target gene is the hsa-miR-4485-5p gene, a congener thereof, a transcript thereof, or a variant or derivative thereof. None of the previously known reports show that change in the expression of the gene or the transcript thereof can serve as a marker for uterine leiomyosarcoma.

The 4th target gene is the hsa-miR-451a gene, a congener thereof, a transcript thereof, or a variant or derivative thereof. None of the previously known reports show that change in the expression of the gene or the transcript thereof can serve as a marker for uterine leiomyosarcoma.

The 5th target gene is the hsa-miR-4635 gene, a congener thereof, a transcript thereof, or a variant or derivative thereof. None of the previously known report shows that change in the expression of the gene or the transcript thereof can serve as a marker for uterine leiomyosarcoma.

The 6th target gene is the hsa-miR-6511b-5p gene, a congener thereof, a transcript thereof, or a variant or derivative thereof. None of the previously known report shows that change in the expression of the gene or the transcript thereof can serve as a marker for uterine leiomyosarcoma.

The 7th target gene is the hsa-miR-191-5p gene, a congener thereof, a transcript thereof, or a variant or derivative thereof. None of the previously known report shows that change in the expression of the gene or the transcript thereof can serve as a marker for uterine leiomyosarcoma.

In one aspect, the present invention relates to a marker for detecting uterine leiomyosarcoma or diagnosing uterine leiomyosarcoma, which comprises at least one selected from the above target nucleic acids. In one aspect, the present invention relates to a marker for differentiating uterine leiomyosarcoma from other diseases, for example, at least one disease selected from the group consisting of uterus adenosarcoma, uterus endometrial stromal sarcoma (ESS), uterine fibroid, and leiomyosarcoma of unknown malignancy (STUMP), which comprises at least one selected from the above target nucleic acids.

In one aspect, the present invention relates to use of at least one selected from the above target nucleic acids for detecting uterine leiomyosarcoma or diagnosing uterine leiomyosarcoma. In one aspect, the present invention relates to use of at least one selected from the above target nucleic acids for differentiating uterine leiomyosarcoma from other diseases, for example, at least one disease selected from the group consisting of uterus adenosarcoma, uterus endometrial stromal sarcoma (ESS), uterine fibroid, and leiomyosarcoma of unknown malignancy (STUMP).

2. Nucleic acid probe or primer for detection of uterine leiomyosarcoma

A nucleic acid probe or primer that can be used for detection of uterine leiomyosarcoma or diagnosis of uterine leiomyosarcoma according to the present invention enables qualitative and/or quantitative measurement of the presence, expression level, or abundance of the following target nucleic acid for uterine leiomyosarcoma: human-derived miR-1246, miR-4430, miR-4485-5p, miR-451a, miR-4635, miR-6511b-5p, miR-191-5p, or any combination thereof, any congener thereof, any transcript thereof, or any variant or derivative thereof.

The expression level of the above target nucleic acid in subjects having uterine leiomyosarcoma may be increased or decreased (hereinafter, also referred to as "increase/decrease"), depending on the kind of the target nucleic acid, compared with healthy subjects, benign disease patients, and subjects having cancer other than uterine leiomyosarcoma. Thus, the kit or device of the present invention can be effectively used for detection of uterine leiomyosarcoma by measuring the expression level of the above target nucleic acid in body fluid derived from a subject (*e.g.*, a human) suspected of having uterine leiomyosarcoma and in body fluids derived from healthy subjects, benign disease patients, and patients with cancer other than uterine leiomyosarcoma and then comparing the measured expression level.

A nucleic acid probe or primer that can be used in the present invention is a nucleic acid probe capable of specifically binding to a polynucleotide consisting of a nucleotide sequence shown in at least one selected from SEQ ID NOs: 1 to 7 or a complementary strand of the polynucleotide, or a primer for amplifying a polynucleotide consisting of a nucleotide sequence shown in at least one selected from SEQ ID NOs: 1 to 7.

In a preferred embodiment of the method of the present invention, the above nucleic acid probe or primer includes any combination of one or more polynucleotides selected from a group of polynucleotides comprising a nucleotide sequence shown in any of SEQ ID NOs: 1 to 43 and a nucleotide sequence derived therefrom by the replacement of u with t, a group of polynucleotides complementary thereto, a group of polynucleotides hybridizing under stringent conditions (described below) to DNA comprising a nucleotide sequence complementary to the former nucleotide sequence, a group of polynucleotides complementary thereto, and a group of polynucleotides comprising 15 or more and preferably 17 or more consecutive nucleotides in the nucleotide sequence of these groups of polynucleotides.

The upper limit of the nucleotide sequence length of these polynucleotides is not particularly limited. When the target nucleic acid is mature miRNA, for example, the number of nucleotides is 30 or less, 28 or less, or 25 or less. When the target nucleic acid is a miRNA precursor, for example, the number of nucleotides is 200 or less, 150 or less, or 120 or less. When the target nucleic acid is isomiR, for example, the number of nucleotides is 40 or less, 35 or less, or 30 or less.

These polynucleotides can be used as nucleic acid probes and primers for detecting target nucleic acids, namely the above uterine leiomyosarcoma markers.

Further, specific examples of the nucleic acid probe or primer that can be used in the present invention include one or more polynucleotides selected from the group consisting of any of the following polynucleotides (a) to (e):

(a) a polynucleotide consisting of a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(b) a polynucleotide comprising a nucleotide sequence shown in any of SEQ ID NOs: 1 to 7;
(c) a polynucleotide consisting of a nucleotide sequence complementary to a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(d) a polynucleotide comprising a nucleotide sequence complementary to a nucleotide sequence shown in any of SEQ ID NOs: 1 to 7 or a nucleotide sequence derived therefrom by the replacement of u with t; and
(e) a polynucleotide hybridizing under stringent conditions to any of the polynucleotides (a) to (d).

The above polynucleotides or fragments thereof used in the present invention may each be DNA or RNA.

The above polynucleotide that can be used in the present invention may be prepared using a general technique such as DNA recombination technology, a PCR method, or a method using an automated DNA/RNA synthesizer.

As the DNA recombination technology or the PCR method, it is possible to use techniques described in, for example, Ausubel et al., Current Protocols in Molecular Biology, John Willey & Sons, US (1993); and Sambrook et al., Molecular Cloning A Laboratory Manual, Cold Spring Harbor Laboratory Press, US (1989).

The human-derived miR-1246, miR-4430, miR-4485-5p, miR-451a, miR-4635, miR-6615b-5p, and miR-191-5p, which are shown in SEQ ID NOs: 1 to 7, are known, and methods for obtaining them are also known, as described above. Therefore, a polynucleotide that can be used as a nucleic acid probe or primer in the present invention can be produced by cloning the gene.

Such a nucleic acid probe or primer may be chemically synthesized using an automated DNA synthesizer. A phosphoramidite process is commonly used for this synthesis, and this process can be used to automatically synthesize a single-stranded DNA with up to about 100 nucleotides in length. The automated DNA synthesizer is commercially available from, for example, Polygen, Inc., ABI, Inc., or Applied BioSystems, Inc.

Alternatively, a polynucleotide of the present invention may be prepared by cDNA cloning. For the cDNA cloning technology, for example, the microRNA Cloning Kit Wako can be utilized.

The sequence of a nucleic acid probe or primer for detection of a polynucleotide consisting of a nucleotide sequence shown in any of SEQ ID NOs: 1 to 7 is not present *in vivo* as an miRNA or a precursor thereof. For example, the nucleotide sequence shown in SEQ ID NO: 1 is generated from the precursor shown in SEQ ID NO: 8. This precursor has a hairpin-like structure as shown in Fig. 1, and the nucleotide sequence shown in SEQ ID NO: 1 has a mismatch sequence. Accordingly, a nucleotide sequence that is completely complementary to the nucleotide sequence shown in SEQ ID NO: 1 is not naturally occurring *in vivo.* Thus, the nucleic acid probe or primer for detecting a nucleotide sequence shown in any of SEQ ID NOs: 1 to 7 may have an artificial nucleotide sequence that is not present *in vivo.*

3. Kit or device for detection of uterine leiomyosarcoma

The present invention provides a kit or device for detection of uterine leiomyosarcoma comprising one or more polynucleotide (which may include any variant, fragment, or derivative) that can be used as a nucleic acid probe or primer for measuring a target nucleic acid as a uterine leiomyosarcoma marker in the present invention. In one embodiment, the kit or device of the present invention can be used for differentiating uterine leiomyosarcoma from other diseases, for example, at least one disease selected from the group consisting of uterus adenosarcoma, uterus endometrial stromal sarcoma (ESS), uterine fibroid, and leiomyosarcoma of unknown malignancy (STUMP).
The target nucleic acid as a uterine leiomyosarcoma marker in the present invention is selected from the group consisting of miR-1246, miR-4430, miR-4485-5p, miR-451a, miR-4635, miR-6511b-5p, and miR-191-5p.
The kit or device of the present invention comprises a nucleic acid capable of specifically binding to the above target nucleic acid as a uterine leiomyosarcoma marker. Preferably, the kit or device comprises one or more polynucleotides selected from the polynucleotides described in Section 2 above or a variant thereof.
Specifically, the kit or device of the present invention may comprise at least one of a polynucleotide comprising (or consisting of) a nucleotide sequence shown in any of SEQ ID NOs: 1 to 7 or a nucleotide sequence derived therefrom by the replacement of u with t, a polynucleotide comprising (or consisting of) a complementary sequence thereof, a polynucleotide hybridizing under stringent conditions to any of the polynucleotides, or a variant or fragment thereof comprising 15 or more consecutive nucleotides of any of the polynucleotide sequences.
The fragment that can be contained in the kit or device of the present invention is, for example, one or more, preferably two or more polynucleotides selected from the group consisting of polynucleotides comprising 15 or more consecutive nucleotides in a nucleotide sequence or a complementary sequence thereof with u replaced with t in a nucleotide sequence shown in any of SEQ ID Nos: 1 to 7.
In a preferred embodiment, the polynucleotide is a polynucleotide consisting of a nucleotide sequence shown in any of SEQ ID NOs: 1 to 7 or a nucleotide sequence derived therefrom by the replacement of u with t, a polynucleotide consisting of a complementary sequence thereof, a polynucleotide hybridizing under stringent conditions to any of the polynucleotides, or a variant comprising 15 or more, preferably 17 or more, and more preferably 19 or more consecutive nucleotides of any of the polynucleotides.
In a preferred embodiment, the fragment may be a polynucleotide comprising 15 or more, preferably 17 or more, and more preferably 19 or more consecutive nucleotides.
In the present invention, the size of the polynucleotide fragment is represented by the number of nucleotides in a range of, for example, 15 or more and less than the total number of consecutive nucleotides, 17 or more and less than the total number of nucleotides, or 19 or more and less than the total number of nucleotides in the nucleotide sequence of each polynucleotide.
Specific examples of the above polynucleotide as target nucleic acid in the kit or device of the present invention include 1 or a combination of 2, 3, 4, 5, 6, 7, 8, 9, 10, or more of the above polynucleotides consisting of nucleotide sequences shown in SEQ ID NOs: 1 to 43 listed in Table 1 above. It should be noted that such polynucleotide are merely examples and all other various possible combinations are within the scope of the present invention.
Examples of a combination of target nucleic acids in a kit or device for distinguishing uterine leiomyosarcoma patients from subjects without uterine leiomyosarcoma, such as healthy subjects, patients with benign bone and soft tissue tumor and benign breast disease, and patients with cancer other than uterine leiomyosarcoma in the present invention include a combination of two or more of the above polynucleotides consisting of nucleotide sequences shown in SEQ ID NOs listed in Table 1. Specifically, any 2 or more, 3 or more, 4 or more, 5 or more, 6 or more, or 7 of the above polynucleotides consisting of nucleotide sequences shown in SEQ ID NOs: 1 to 7 can be combined. In particular, combinations containing a polynucleotide consisting of SEQ ID No: 1 or combinations containing a polynucleotide consisting of SEQ ID No: 2 are preferable.
The kit or device of the present invention may comprise, in addition to the above-described polynucleotide in the present invention (which may include any variant, fragment, or derivative), a polynucleotide known to be able to detect uterine leiomyosarcoma or a polynucleotide that will be discovered in the future.
The kit or device of the present invention may comprise, in addition to the above-described polynucleotides according to the present invention, an antibody (or antibodies) for measuring a known marker for detection of uterine leiomyosarcoma, such as LDH.
These polynucleotides, variants thereof, or fragments thereof contained in the kit of the present invention may be packaged in different containers either individually or in any combination.
The kit of the present invention may comprise a kit for extracting nucleic acids (e.g., total RNA) from body fluids, cells, or tissues, a fluorescent material for labeling, an enzyme and a medium for nucleic acid amplification, an instruction manual, etc.
The device of the present invention may be a device for measuring a cancer marker, in which nucleic acids such as the above-described polynucleotide, variant, derivative, or fragment thereof according to the present invention are, for example, bonded or attached onto a solid phase. Examples of the material for the solid phase include plastics, paper, glass, and silicone. The material for the solid phase is preferably a plastic from the viewpoint of easy processability. The solid phase has any shape and is, for example, square, round, reed-shaped, or film-shaped. The device of the present invention includes, for example, a device for measurement by a hybridization technique. Specific examples thereof include blotting devices and nucleic acid arrays (e.g., microarrays, DNA chips, and RNA chips).

The nucleic acid array technique involves bonding or attaching the nucleic acids one by one by a method, such as a method of spotting the nucleic acids using a high-density dispenser referred to as a spotter or arrayer onto the surface of the solid phase that has been subjected to surface treatment such as coating with L-lysine or introduction of a functional group (e.g., an amino group or a carboxyl group), according to need, a method of spraying the nucleic acids onto the solid phase using an inkjet which injects very small liquid droplets by a piezoelectric element or the like through a nozzle, or a method of sequentially synthesizing nucleotides on the solid phase, to prepare an array such as a chip and measuring target nucleic acids via hybridization using such array.

The kit or device of the present invention comprises a nucleic acid capable of specifically binding to at least one, preferably at least two, most preferably at least three to all polynucleotides of the above uterine leiomyosarcoma marker miRNAs, or each of complementary strands of such polynucleotides.

The kit or device of the present invention can be used for detecting uterine leiomyosarcoma as described in Section 4 below.

4. Method for detecting uterine leiomyosarcoma

The present invention further relates to a method for detecting uterine leiomyosarcoma, comprising measuring, *in vitro,* at least one expression level (*e.g.*, expression profile) of a gene selected from the group consisting of miR-1246, miR-4430, miR-4485-5p, miR-451a, miR-4635, miR-6511b-5p, and miR-191-5p in a sample and evaluating, *in vitro,* whether or not a subject has uterine leiomyosarcoma using the measured expression level (and optionally a control expression level in a healthy subject measured in the same manner). This method comprises, for example, using the expression level of the gene in a sample collected from a subject suspected of having uterine leiomyosarcoma (*e.g.*, blood, serum, or plasma) and the control gene expression level in a sample collected from a test subject without uterine leiomyosarcoma (*e.g.*, blood, serum, or plasma) (*e.g.*, these gene expression levels may be compared). When a difference is observed in the expression level of the target nucleic acid in the sample, the subject can be determined to have uterine leiomyosarcoma.

In one embodiment, the method of the present invention can be a method for differentiating uterine leiomyosarcoma from other diseases, for example, at least one disease selected from the group consisting of uterus adenosarcoma, uterus endometrial stromal sarcoma (ESS), uterine fibroid, and leiomyosarcoma of unknown malignancy (STUMP).

The method of the present invention enables early diagnosis of uterine leiomyosarcoma in a low-invasive manner with high sensitivity and specificity. This allows early treatment and improved prognosis and further enables observation of disease exacerbation and observation of effectiveness of surgical, radiotherapeutic, and chemotherapeutic treatment.

For the method for extracting the uterine leiomyosarcoma-derived gene from the sample such as blood, serum, or plasma according to the present invention, it is particularly preferable to add a reagent for RNA extraction in 3D-Gene (registered trademark) RNA extraction reagent from liquid sample kit (Toray Industries, Inc., Japan) to prepare a sample. Alternatively, a general acidic phenol method (an acid guanidinium-phenol-chloroform (AGPC) method) may be used, or Trizol (registered trademark) (Life Technologies Corp.) may be used. Alternatively, a reagent for RNA extraction containing acidic phenol, such as Trizol (Life Technologies Corp.) or Isogen (Nippon Gene Co., Ltd., Japan), may be added to prepare a sample. In addition, a kit such as miRNeasy (registered trademark) Mini Kit (Qiagen) may be used, although the method is not limited thereto.

The present invention also provides use of a uterine leiomyosarcoma-derived miRNA gene in a sample from a subject for *in vitro* detection of an expression product thereof.

A technique for carrying out the method of the present invention is not limited. For example, the kit or device of the present invention (comprising the above nucleic acid that can be used in the present invention) as described in Section 3 above may be used. The method of the present invention involves the use of the kit or device comprising a single polynucleotide or any possible combination of polynucleotides that can be used in the present invention, as described above.

In the detection or (genetic) diagnosis of uterine leiomyosarcoma according to the present invention, a polynucleotide contained in the kit or device of the present invention can be used as a probe or a primer. When using polynucleotide as a primer, TaqMan (registered trademark) MicroRNA Assays (Life Technologies Corp.), miScript PCR System (Qiagen), or the like can be used, although the present invention is not limited thereto.

In the method of the present invention, measurement of the gene expression levels can be performed according to a conventional technique known in the art that specifically detects particular genes. Examples thereof include hybridization techniques, such as Northern blotting, Southern blotting, *in situ* hybridization, Northern hybridization, and Southern hybridization, a quantitative amplification technique such as quantitative RT-PCR, and a method involving the use of a next-generation sequencer. A body fluid such as blood, serum, plasma, or urine from a subject is collected as a sample to be assayed depending on the type of the detection method to be used. Alternatively, total RNA prepared from such a body fluid by the method described above may be used, and various polynucleotides including cDNA prepared from the RNA may be used.

The method of the present invention is useful for diagnosis of uterine leiomyosarcoma or detection of the presence or absence of uterine leiomyosarcoma. Specifically, detection of uterine leiomyosarcoma according to the present invention can be performed by detecting *in vitro* an expression level of a gene, which is detected using, for example, the nucleic acid probe or primer contained in the kit or the device according to the present invention, in a sample such as blood, serum, plasma, or urine from a subject suspected of having uterine leiomyosarcoma. If the expression level of a polynucleotide consisting of a nucleotide sequence shown in at least one of SEQ ID NOs: 1 to 7 in a sample such as blood, serum, plasma, or urine of a subject suspected of having uterine leiomyosarcoma, is statistically significantly different (for example, lower) than an expression level thereof in a sample such as blood, serum, plasma, or urine of a subject without uterine leiomyosarcoma, the former subject can be evaluated as having uterine leiomyosarcoma.

Regarding the method of the present invention, the method for detecting the presence or absence of uterine leiomyosarcoma in a sample from a subject comprises collecting a body fluid such as blood, serum, plasma, or urine of the subject, and measuring the expression level of the target gene (or target nucleic acid) contained therein using one or more polynucleotides (including a variant, fragment, or derivative) selected from the polynucleotide group according to the present invention, thereby evaluating the presence or absence of uterine leiomyosarcoma or detecting uterine leiomyosarcoma.

The method for detecting uterine leiomyosarcoma according to the present invention can be performed in combination with other uterine leiomyosarcoma-related tests, such as rectal examination, transrectal ultrasonography of the prostate, biopsy, or imaging tests, such as CT, MRI, and bone scintigraphy, aimed at, for example, more precise diagnosis of uterine leiomyosarcoma in a uterine leiomyosarcoma patient. In addition, the method of the present invention can be performed prior to such testing in order to evaluate the necessity for performance of such uterine leiomyosarcoma-related tests.

The method for detecting uterine leiomyosarcoma according to the present invention can be employed for evaluation or diagnosis as to presence or absence of remediation of uterine leiomyosarcoma or an extent of remediation when, for example, a uterine leiomyosarcoma patient is subjected to treatment or remediation of uterine leiomyosarcoma by means of uterine leiomyosarcoma-related treatment techniques that are known or in the development stage (examples of such treatment techniques include, but are not limited to: radiation treatment techniques, such as x-ray, proton beam, and heavy particle therapy, and high-dose rate interstitial radiation and permanent brachytherapy among radiation treatment techniques; hormonal treatment techniques using luteinizing hormone releasing factor (LH-RH) agonists, such as goserelin acetate and leuprorelin acetate, anti-androgen drugs, such as chlormadinone acetate, flutamide, picartamide, enzalutamide, and abiraterone acetate, LH-RH antagonists, such as degarelix acetate, and estrogen, such as ethinyl estradiol; chemotherapy techniques using drugs, such as docetaxel hydrate, cabazitaxel, and estramustine phosphate sodium hydrate; and use of such treatment techniques in adequate combination).

Another aspect of the present invention provides a method for treatment of uterine leiomyosarcoma. Specifically, the method for treatment of uterine leiomyosarcoma according to the present invention comprises a step of performing the treatment of uterine leiomyosarcoma (radiation treatment, hormonal treatment, chemotherapy, and use of such treatment techniques in combination) on a subject who is determined to have uterine leiomyosarcoma as a result of the method of detection described above.

The method of the present invention may comprise, for example, the following steps (a), (b), and (c):

(a) contacting, *in vitro,* a sample from a subject with a polynucleotide contained in the kit or device of the present invention;
(b) measuring an expression level of a target nucleic acid in the sample using the polynucleotide as a nucleic acid probe or primer; and
(c) evaluating the presence or absence of uterine leiomyosarcoma (cells) in the subject on the basis of the measurement results in step (b).

In one embodiment, the present invention provides a method for detecting uterine leiomyosarcoma comprising measuring an expression level of a target nucleic acid in a sample from a subject using a nucleic acid capable of specifically binding to at least one, and preferably at least two polynucleotides selected from the group consisting of miR-1246, miR-4430, miR-4485-5p, miR-451a, miR-4635, miR-6511b-5p, and miR-191-5p, or a complementary strand of the polynucleotide; and evaluating *in vitro* whether or not the subject has uterine leiomyosarcoma using the measured expression level and a control expression level in a test subject without uterine leiomyosarcoma measured in the same manner.

The term "evaluating" as used herein is evaluation support based on the results of *in vitro* examination, which is not physician’s judgment.

In the method of the present invention, specifically, miR-1246 is hsa-miR-1246, miR-4430 is hsa-miR-4430, miR-4485-5p is hsa-miR-4485-5p, miR-451a is hsa-miR-451a, miR-4635 is hsa-miR-4635, miR-6511b-5p is hsa-miR-6511b-5p, and miR-191-5p is hsa-miR-191-5p, as described above.

Examples of the sample used in the method of the present invention can include samples prepared from living tissues (preferably prostate tissues, renal pelvis, or urinary tract tissues) or body fluids such as blood, serum, plasma, and urine from subjects. Specifically, an RNA-containing sample prepared from the tissue, a polynucleotide-containing sample further prepared therefrom, a body fluid such as blood, serum, plasma, or urine, a portion or the whole of a living tissue collected from the subject via biopsy or other means, a living tissue excised by surgery, or the like can be used, and the sample for measurement can be prepared therefrom.

The term "subject" used herein refers to a mammal, such as a human, a monkey, a mouse, or a rat, with a human being preferable, although the subject is not limited thereto.

The steps of the method of the present invention can be changed depending on the type of the sample to be measured.

When using RNA as an analyte, the method for detecting uterine leiomyosarcoma (cells) can comprise, for example, the following steps (a), (b), and (c):

(a) binding RNA prepared from a sample from a subject (wherein, for example, the 3’ end of the RNA may be polyadenylated for quantitative RT-PCR in step (b) or any sequence may be added to one or both ends of the RNA by ligation) or a complementary polynucleotide (cDNA) transcribed from the RNA to a polynucleotide in the kit of the present invention;

(b) measuring the sample-derived RNA or the cDNA synthesized from the RNA, which has been bound to the polynucleotide, by hybridization using the polynucleotide as a nucleic acid probe or by quantitative RT-PCR using the polynucleotide as a primer; and

(c) evaluating the presence or absence of uterine leiomyosarcoma (or the uterine leiomyosarcoma-derived gene) on the basis of the measurement results of step (b).

For example, various hybridization methods can be used for measuring the expression level of a target gene according to the present invention. Examples of hybridization methods that can be used include Northern blotting, Southern blotting, DNA chip analysis, *in situ* hybridization, Northern hybridization, and Southern hybridization. PCR such as quantitative RT-PCR or next-generation sequencing can also be used in combination with or alternative to the hybridization method.

When using Northern blotting, the presence or absence of expression of each gene or the expression level thereof in the RNA can be detected or measured using, for example, the nucleic acid probe that can be used in the present invention. A specific example thereof is a method, which comprises labeling the nucleic acid probe (or a complementary strand) with a radioisotope ($^{32}P$, $^{33}P$, $^{35}S$, etc.) or a fluorescent material, hybridizing the labeled product with the living tissue-derived RNA from a subject, which is transferred to a nylon membrane in accordance with a conventional technique, and then detecting and measuring a signal derived from the label (a radioisotope or fluorescent material) on the formed DNA/RNA double strand using a radiation detector (e.g., BAS-1800 II, Fujifilm Corp.) or a fluorescence detector (e.g., STORM 865, GE Healthcare Japan Corp.).

When using quantitative RT-PCR, the presence or absence of expression of each gene or the expression level thereof in the RNA can be detected or measured using, for example, the primer that can be used in the present invention. A specific example thereof is a method, which comprises collecting the living tissue-derived RNA from a subject, polyadenylating the 3’-end, preparing cDNAs from the polyadenylated RNA in accordance with a conventional method, performing PCR in accordance with a conventional method by hybridizing a pair of primers (consisting of a plus strand and a reverse strand each binding to the cDNA) that could be contained in the kit or device for detection of the present invention with the cDNA, so as to amplify the region of each target gene marker with the cDNA as a template, and detecting the obtained single-stranded or double-stranded DNA. The method for detecting the single-stranded or double-stranded DNA can include a method of performing the PCR using the primers labeled in advance with a radioisotope or a fluorescent material, a method of electrophoresing the PCR product on an agarose gel and staining the double-stranded DNA with ethidium bromide or the like for detection, and a method of transferring the produced single-stranded or double-stranded DNA to a nylon membrane or the like in accordance with a conventional method and hybridizing the single-stranded or double-stranded DNA with a labeled nucleic acid probe for detection.

When using quantitative RT-PCR, a commercially available kit for measurement specially designed for quantitative measurement of miRNA, such as TaqMan (registered trademark) MicroRNA Assays (Life Technologies Corp.),

LNA (registered trademark)-based MicroRNA PCR (Exiqon), or Ncode (registered trademark) miRNA qRT-PCT kit (Invitrogen Corp.), may be used.

When using nucleic acid array analysis, for example, an RNA chip or a DNA chip in which the kit or device for detection in the present invention is attached as nucleic acid probes (single-stranded or double-stranded) to a substrate (solid phase) is used. Regions comprising the nucleic acid probes attached thereto are referred to as probe spots, and regions comprising no nucleic acid probes attached thereto are referred to as blank spots. A group of genes immobilized on a solid-phase substrate is generally referred to as a nucleic acid chip, a nucleic acid array, a microarray, or the like. The DNA or RNA array includes a DNA or RNA macroarray and a DNA or RNA microarray. In the present specification, the term "chip" includes these arrays. 3D-Gene (registered trademark) Human miRNA Oligo chip (Toray Industries, Inc., Japan) can be used as the DNA chip, although the DNA chip is not limited thereto.

Examples of the measurement using the DNA chip can include, but are not limited to, a method of detecting and measuring a signal derived from the label on the kit or device for detection using an image detector (e.g., Typhoon 9410, GE Healthcare, and 3D-Gene (registered trademark) scanner, Toray Industries, Inc., Japan).

Under the "stringent conditions" used herein, as described above, a nucleic acid probe hybridizes to its target sequence to a detectably larger extent (e.g., a measurement value equal to or larger than "a mean of background measurement values + a standard error of the background measurement values $\times$ 2") than that for other sequences.

The stringent conditions are defined by hybridization and subsequent washing. Examples of the hybridization conditions include, but are not limited to, 30°C to 60°C for 1 to 24 hours in a solution containing SSC, a surfactant, formamide, dextran sulfate, a blocking agent, etc. In this context, 1 $\times$ SSC is an aqueous solution (pH 7.0) containing 150 mM sodium chloride and 15 mM sodium citrate. The surfactant includes, for example, SDS (sodium dodecyl sulfate), Triton, or Tween. The hybridization conditions more preferably comprise 3-10 $\times$ SSC and 0.1-1% SDS. Examples of the conditions for the washing, following the hybridization, which is another condition to define the stringent conditions, can include conditions comprising continuous washing at 30°C in a solution containing 0.5 $\times$ SSC and 0.1% SDS, at 30°C in a solution containing 0.2 $\times$ SSC and 0.1% SDS, and at 30°C in a 0.05 $\times$ SSC solution. It is desirable that the complementary strand remain hybridized to a target plus strand even washed under such conditions. Specific examples of such complementary strand can include a strand consisting of a nucleotide sequence in a completely complementary relationship with the nucleotide sequence of the target plus (+) strand, and a strand consisting of a nucleotide sequence having at least 80%, preferably at least 85%, more preferably at least 90%, or at least 95% homology (identity) to the strand.

Other examples of the "stringent conditions" for the hybridization are described in, for example, Sambrook, J. & Russel, D., Molecular Cloning, A LABORATORY MANUAL, Cold Spring Harbor Laboratory Press, published on January 15, 2001, Vol. 1, 7.42 to 7.45 and Vol. 2, 8.9 to 8.17, and can be used in the present invention.

Examples of the conditions for carrying out PCR using polynucleotide fragments in the kit of the present invention as primers include treatment for approximately 15 seconds to 1 minute at 5 to 10°C plus a Tm value calculated from the sequences of the primers using a PCR buffer having a composition such as 10 mM Tris-HCL (pH 8.3), 50 mM KCl, and 1 to 2 mM $MgCl_2$. Examples of the method for calculating such a Tm value include Tm value = 2 $\times$ (the number of adenine residues + the number of thymine residues) + 4 $\times$ (the number of guanine residues + the number of cytosine residues).

In the method of the present invention, measurement of the target gene expression level may be performed with a sequencer, in addition to the hybridization methods described above. When using a sequencer, any of DNA sequencers of the first generation based on the Sanger method, the second generation with a shorter read size, and the third generation with a longer read size can be used (referred to as the "next-generation sequencer," including sequencers of the second generation and the third generation herein). A commercially available measurement kit specially designed for measurement of miRNA may be used with the use of, for example, Miseq, Hiseq, or NexSeq (Illumina, Inc.), Ion Proton, Ion PGM, or Ion S5/S5 XL (Thermo Fisher Scientific Inc.), PacBio RS II or Sequel (Pacific Biosciences of California, Inc.), or, when using a Nanopore sequencer, MinION (Oxford Nanopore Technologies Ltd.).

Next-generation sequencing is a method of obtaining sequence information using a next-generation sequencer, which is characterized in that a significantly larger number of sequencing reactions can be simultaneously performed, compared with the Sanger method (e.g., Rick Kamps et al., Int. J. Mol. Sci., 2017, 18(2), p. 308 and Int. Neurourol. J., 2016, 20 (Suppl.2), S76-83). Examples of next-generation sequencing steps for miRNA include, but are not limited to, the following steps: at the outset, adaptor sequences having predetermined nucleotide sequences are attached, and all RNAs are reverse-transcribed into cDNAs before or after attachment of the sequences. After the reverse transcription, cDNAs derived from specific target miRNAs may be amplified or concentrated by PCR or other means or with a probe or the like, so as to analyze the target miRNA before sequencing steps. While details of subsequent sequencing steps vary depending on the type of a next-generation sequencer, typically, a sequencing reaction is performed by linking the sequence of interest to a substrate via an adaptor sequence and further using the adaptor sequence as a priming site. Concerning the details of the sequencing reaction, a reference may be made to, for example, Rick Kamps *et al.* (see supra). In the end, the data are outputted. This step provides a collection of sequence information (reads) obtained by the sequencing reaction. For example, next-generation sequencing enables identification of a target miRNA based on the sequence information and measurement of the expression level thereof based on the number of reads having the sequences of the target miRNA.

In the present invention, gene expression levels can be calculated via statistical treatment described in, for example, Statistical analysis of gene expression microarray data (Speed T., Chapman and Hall/CRC), and A beginner's guide: Microarray gene expression data analysis (Causton H. C. et al., Blackwell publishing), although the method of calculation is not limited thereto. For example, 2 times, preferably 3 times, and more preferably 6 times the standard deviation of the measurement values of the blank spots are added to the average measurement value of the blank spots on the DNA chip, and probe spots having a signal value equal to or larger than the resulting value can be regarded as detection spots. Alternatively, the average measurement value of the blank spots is regarded as a background and can be subtracted from the measurement values of the probe spots to determine gene expression levels. A missing value for a gene expression level can be excluded from the analyte, preferably replaced with the smallest value of the gene expression level in each DNA chip, or more preferably replaced with a value obtained by subtracting 0.1 from a logarithmic value of the smallest value of the gene expression level. In order to eliminate low-signal genes, only a gene having a gene expression level of $2^6$, preferably $2^8$, and more preferably $2^{10}$ or larger in 20% or more, preferably 50% or more, and more preferably 80% or more of the number of measurement samples can be selected as the analyte. Examples of the normalization of the gene expression level include, but are not limited to, global normalization and quantile normalization (Bolstad, B.M. et al., 2003, Bioinformatics, Vol. 19, pp. 185-193). Further examples of methods include: a method in which endogenous genes exhibiting constitutive expression levels regardless of samples are identified as control samples and used for normalization; and a method in which a given amount of particular nucleic acids are added externally and the amount thereof is used for normalization.

The present invention also provides a method for detecting uterine leiomyosarcoma (or assisting the detection thereof) in a subject comprising: measuring target gene expression levels in a sample from the subject; and assigning the expression levels of the target genes in the sample from the subject to a discriminant (discriminant function), which is prepared using gene expression levels of a sample from a subject (or a patient) known to have uterine leiomyosarcoma and a sample from a test subject without uterine leiomyosarcoma, as a training sample, and is capable of distinguishing the presence or absence of uterine leiomyosarcoma, thereby evaluating the presence or absence of uterine leiomyosarcoma.

Specifically, the present invention further provides the method comprising: a first step of measuring *in vitro* expression levels of target genes in a plurality of samples from subjects known to be with uterine leiomyosarcoma and/or without uterine leiomyosarcoma; a second step of preparing a discriminant formula using the measured expression levels of the target genes obtained in the first step as training samples; a third step of measuring *in vitro* the expression levels of the target genes in a sample from the subject in the same manner as in the first step; and a fourth step of assigning the measured expression levels of the target genes obtained in the third step to the discriminant formula obtained in the second step, and determining or evaluating whether or not the subject has uterine leiomyosarcoma on the basis of the results obtained from the discriminant formula. The above target genes are those that can be detected, for example, by the polynucleotides, the polynucleotides contained in the kit or chip, and variants thereof or fragments thereof.

The discriminant formulae herein can be prepared with the use of any discriminant analysis methods, based on which a discriminant formula that distinguishes the presence or absence of uterine leiomyosarcoma can be prepared, such as Fisher's discriminant analysis, nonlinear discriminant analysis based on the Mahalanobis' distance, neural network, Support Vector Machine (SVM), logistic regression analysis (especially, logistic regression analysis using the LASSO (Least Absolute Shrinkage and Selection Operator)), k-nearest neighbor method, or decision tree, although the analysis method is not limited to these specific examples.

When a clustering boundary is a straight line or a hyperplane, the linear discriminant analysis is a method for determining the belonging of a cluster using Formula 1 as a discriminant formula. In Formula 1, "x" represents an explanatory variable, w represents a coefficient of the explanatory variable, and wo represents a constant term.

$$f(x) = w_0 + \sum_{i=1}^{n} w_i x_i \qquad \text{Formula 1}$$

Values obtained from the discriminant formula are referred to as discriminant scores. The measurement values of a newly offered data set can be assigned as explanatory variables to the discriminant formula to determine clusters by the signs of the discriminant scores.

The Fisher's discriminant analysis, a type of linear discriminant analysis, is a dimensionality reduction method for selecting a dimension suitable for discriminating classes, and constructs a highly discriminating synthetic variable by focusing on the variance of the synthetic variables and minimizing the variance of data having the same label (Venables, W. N. et al., Modern Applied Statistics with S. Fourth edition. Springer., 2002). In the Fisher's discriminant analysis, direction w of projection is determined so as to maximize Formula 2. In this formula, μ represents an average input, $n_g$ represents the number of data belonging to class g, and $\mu_g$ represents an average input of the data belonging to class g. The numerator and the denominator are the interclass variance and the intraclass variance, respectively, when each of data is projected in the direction of the vector w. Discriminant coefficient wi is determined by maximizing this ratio (Takafumi Kanamori et al., "Pattern Recognition," KYORITSU SHUPPAN CO., LTD. (Tokyo, Japan) (2009); Richard O. et al., Pattern Classification, Second Edition., Wiley-Interscience, 2000).

$$J(w)=\frac{\sum_{g=1}^{G} n_g\left(w^T\mu_g - w^T\mu\right)\left(w^T\mu_g - w^T\mu\right)^T}{\sum_{g=1}^{G}\sum_{i:y_i=g}\left(w^T x_i - w^T\mu_g\right)\left(w^T x_i - w^T\mu_g\right)} \qquad \text{Formula 2}$$

*subject to* $\mu=\sum_{i=1}^{n}\frac{x_i}{n}$, $\mu_g=\sum_{i:u_i=g}^{n}\frac{x_i}{n_g}$

The Mahalanobis' distance is calculated according to Formula 3 in consideration of data correlation and can be used as nonlinear discriminant analysis for determining a cluster in which a data point belongs to, based on a short Mahalanobis' distance from the data point to that cluster. In Formula 3, μ represents a central vector of each cluster, and $S^{-1}$ represents an inverse matrix of the variance-covariance matrix of the cluster. The central vector is calculated from explanatory variable x, and an average vector, a median value vector, or the like can be used.

$$D(x,\mu)=\left\{(x-\mu)^t S^{-1}(x-\mu)\right\}^{\frac{1}{2}} \qquad \text{Formula 3}$$

SVM is a discriminant analysis method devised by V. Vapnik (The Nature of Statistical Leaning Theory, Springer, 1995). Particular data points of a data set having known classes are defined as explanatory variables, and classes are defined as objective variables. A boundary plane referred to as a hyperplane for correctly classifying the data set into the known classes is determined, and a discriminant formula for data classification is determined using the boundary plane. Then, the measurement values of a newly offered data set can be assigned as explanatory variables to the discriminant formula to determine classes. In this respect, the result of the discriminant analysis may be classes, a probability of being classified into appropriate classes, or the distance from the hyperplane. In SVM, a method of nonlinearly converting a feature vector to a high dimension and performing linear discriminant analysis in the space is known as a method for tackling nonlinear problems. An expression in which an inner product of two factors in a nonlinearly mapped space is expressed only by inputs in their original spaces is referred to as "kernel." Examples of the kernel can include a linear kernel, an RBF (Radial Basis Function) kernel, and a Gaussian kernel. While highly dimensional mapping is performed according to the kernel, the optimum discriminant formula, *i.e.*, a discriminant formula, can be actually constructed by merely calculating the kernel while avoiding calculation of features in the mapped space (e.g., Hideki Aso et al., Frontier of Statistical Science 6 "Statistics of pattern recognition and learning - New concepts and approaches," Iwanami Shoten, Publishers (Tokyo, Japan) (2004); Nello Cristianini et al., Introduction to SVM, Kyoritsu Shuppan Co., Ltd. (Tokyo, Japan) (2008)).

C-support vector classification (C-SVC), a type of SVM, comprises preparing a hyperplane by training a data set with the explanatory variables of two groups and classifying an unknown data set into either of the groups (C. Cortes et al., 1995, Machine Learning, Vol. 20, pp. 273-297).

Exemplary calculation of the C-SVC discriminant formula that can be used in the method of the present invention will be given below. First, all subjects are divided into two groups, *i.e.*, a group of uterine leiomyosarcoma patients and a group of test subjects without uterine leiomyosarcoma. For example, a prostate tissue test can be used as a reference of determining whether or not a subject has uterine leiomyosarcoma.

Next, a data set consisting of comprehensive gene expression levels of serum-derived samples of the two divided groups (hereinafter, this data set is referred to as a training cohort) is prepared, and a C-SVC discriminant formula is determined using genes found to differ clearly in their gene expression levels between the two groups as explanatory variables and this grouping as objective variables (e.g., -1 and +1). An optimizing objective function is represented by Formula 4, wherein "e" represents all input vectors, "y" represents an objective variable, "a" represents a Lagrange's undetermined multiplier vector, "Q" represents a positive definite matrix, and "C" represents a parameter for adjusting constrained conditions.

$$\min_{a} \quad \frac{1}{2}a^T Qa - e^T a \qquad \text{Formula 4}$$

*subject to* $y^T a = 0,\ 0 \le a_i \le C,\ i = 1,\ldots, l,$

Formula 5 is a finally obtained discriminant formula, and a group to which the data point belongs can be determined on the basis of the sign of a value obtained according to the discriminant formula. In this formula, "x" represents a support vector, "y" represents a label indicating the belonging of a group, "a" represents the corresponding coefficient, "b" represents a constant term, and "K" represents a kernel function.

$$f(x) = \mathrm{sgn}\left(\sum_{i=1}^{l} y_i a_i K(x_i, x) + b\right) \qquad \text{Formula 5}$$

For example, an RBF kernel defined by Formula 6 can be used as the kernel function. In this formula, "x" represents a support vector, and "γ" represents a kernel parameter for adjusting the complexity of the hyperplane.

$$K(x_i, x_j) = \exp\left(-r\|x_i - x_j\|^2\right), \quad r < 0 \qquad \text{Formula 6}$$

Logistic regression is a multivariate analysis method in which one category variable (binary variable) is used as an objective variable to predict the probability of occurrence using multiple explanatory variables, which is represented by Formula 7 below.

$$\mathrm{logit}(\mathrm{prob}(y_i = 1)) = \beta_0 + \sum_{j=1}^{p} \beta_j \chi_j \qquad \text{Formula 7}$$

The LASSO (Least Absolute Shrinkage and Selection Operator) method is one of techniques for selecting and adjusting variables when multiple variables are observed, which was proposed by Tibshirani (Tibshirani R., 1996, J. R. Stat. Soc. Ser. B, Vol. 58, pp. 267-88). The LASSO method is characterized in that penalties are imposed upon estimation of regression coefficients, so that overfitting to a model is suppressed and some of the regression coefficients are then estimated to be 0. In logistic regression using the LASSO method, regression coefficients are estimated so as to maximize a log-likelihood function represented by Formula 8.

$$\frac{1}{N}\sum_{i=1}^{N}\left(y_i\left(\beta_0 + \chi_j^T\beta\right) - \log\left(1 + e^{\left(\beta_0 + \chi_j^T\beta\right)}\right)\right) - \lambda\sum_{j=1}^{p}|\beta_j| \qquad \text{Formula 8}$$

The value y of the discriminant formula obtained in analysis by the LASSO method is assigned to the logistic function represented by Formula 9 below, and the group to which the subject belongs can be determined on the basis of the obtained value.

$$\mathrm{Exp}(y)/(1+\exp(y)) \qquad \text{Formula 9}$$

The method of the present invention can comprise, for example, the following steps (a), (b), and (c):

(a) measuring an expression level of a target gene in samples already known to be from uterine leiomyosarcoma patients and to be from subjects without uterine leiomyosarcoma, using polynucleotide, a kit, or a DNA chip for detection according to the present invention;
(b) preparing the discriminant formulae 1 to 3, 5, 6, and 9 described above based on the expression level measured in step (a); and
(c) measuring an expression level of the target gene in a sample from a subject using the polynucleotide, the kit, or the device (*e.g.*, a DNA chip) for diagnosis (detection) according to the present invention, assigning the obtained measurement value to the discriminants prepared in step (b), and determining or evaluating whether or not the subject has uterine leiomyosarcoma on the basis of the obtained results or evaluating the expression level derived from a uterine leiomyosarcoma patient in comparison with a control from a test subject without uterine leiomyosarcoma.

In the discriminant formulae 1 to 3, 5, 6, and 9, "x" represents an explanatory variable and includes a value obtained by measuring a polynucleotide selected from the polynucleotides described in Section 2 above, or a fragment thereof. Specifically, the explanatory variable of the present invention for discriminating a uterine leiomyosarcoma patient from a test subject without uterine leiomyosarcoma is a gene expression level selected from, for example, the gene expression level in sera of a uterine leiomyosarcoma patient and a test subject without uterine leiomyosarcoma as measured by any DNA comprising 15 or more consecutive nucleotides in the nucleotide sequence shown in any of SEQ ID NOs: 1 to 7 or in a complementary sequence thereof.

As described above, the method for determining or evaluating whether or not a subject has uterine leiomyosarcoma using a sample from the subject necessitates the use of a discriminant formula employing one or more gene expression levels as an explanatory variable. In order to improve the accuracy of the discriminant formula employing only a single gene expression level, in particular, it is necessary to use a gene exhibiting a clear difference in expression levels between two groups consisting of a group of uterine leiomyosarcoma patients and a group of test subjects without uterine leiomyosarcoma, in a discriminant formula.

Specifically, the gene that is used for an explanatory variable of a discriminant formula is preferably determined as follows. First, comprehensive gene expression levels of a group of uterine leiomyosarcoma patients and comprehensive gene expression levels of a group of test subjects without uterine leiomyosarcoma, both of which are in a training cohort, are used as a data set, and the degree of difference in the expression level of each gene between the two groups is obtained with the use of, for example, the P value of a parametric analysis such as the t-test, the P value of a nonparametric analysis such as the Mann-Whitney's U test, or the P value of the Wilcoxon test.

The gene can be regarded as being statistically significant when the critical rate (significance level) as the P value obtained by the test is smaller than, for example, 5%, 1%, or 0.01%.

In order to correct an increased probability of type I error attributable to the repetition of test, a method known in the art, such as the Bonferroni or Holm method, can be used for the correction (e.g., Yasushi Nagata et al., "Basics of statistical multiple comparison methods," Scientist Press Co., Ltd. (Tokyo, Japan) (2007)). In the case of the Bonferroni correction, for example, the P value obtained by a test is multiplied by the number of repetitions of test, *i.e.*, the number of genes used in the analysis, and the obtained value is compared with a desired significance level, so that a possibility of type I error caused in the entire tests can be suppressed.

Instead of the test, the absolute value (fold change) of an expression ratio of a median value of each gene expression level between gene expression levels of a group of uterine leiomyosarcoma patients and gene expression levels of a group of test subjects without uterine leiomyosarcoma may be calculated to select a gene that is used for an explanatory variable in a discriminant formula. Alternatively, ROC curves may be prepared using gene expression levels of a group of uterine leiomyosarcoma patients and a group of test subjects without uterine leiomyosarcoma, and a gene that is used for an explanatory variable in a discriminant formula can be selected on the basis of an AUROC value.

Next, a discriminant formula that can be calculated by various methods described above is prepared using any number of genes exhibiting a large difference in their gene expression levels determined here. Examples of the method for constructing a discriminant that produces the largest discrimination accuracy include a method of constructing a discriminant formula in every combination of genes that satisfy the significance level being P value, and a method of repetitively evaluating the genes for use in the preparation of a discriminant formula while increasing the number of genes one by one in descending order of difference in gene expression level (Furey TS. et al., 2000, Bioinformatics., Vol. 16, pp. 906-14). To the discriminant formula, the gene expression level of another independent uterine leiomyosarcoma patient or a test subject without uterine leiomyosarcoma is assigned as an explanatory variable to calculate discrimination results of the group to which the independent uterine leiomyosarcoma patient or the test subject without uterine leiomyosarcoma belongs. Specifically, the set of genes for diagnosis found and the discriminant formula constructed using the set of genes for diagnosis can be evaluated in an independent sample cohort to find more universal set of genes for diagnosis that can detect uterine leiomyosarcoma and a more universal method for discriminating uterine leiomyosarcoma.

When preparing a discriminant formula using expression levels of a plurality of genes as explanatory variables, it is not necessary to select a gene exhibiting a clear difference in expression levels between the group of uterine leiomyosarcoma patients and the group of test subjects without uterine leiomyosarcoma as described above. Even if there is no clear difference in expression levels of individual genes, specifically, a discriminant formula with high discriminant performance may be obtained with the use of expression levels of a plurality of genes in combination. Thus, a method of directly searching for a discriminant formula with high discriminant performance can be employed without selecting the gene to be employed in the discriminant formula in advance.

The split-sample method is preferably used for evaluating the performance (generality) of the discriminant formula. Specifically, a data set is divided into a training cohort and a validation cohort, and gene selection by a statistical test and preparation of a discriminant formula are performed in the training cohort. Accuracy, sensitivity, and specificity are calculated using the results of discrimination of a validation cohort according to the discriminant formula and a true group to which the validation cohort belongs, to thereby evaluate the performance of the discriminant. On the other hand, gene selection by a statistical test and preparation of a discriminant formula may be performed using all the samples without dividing a data set, and accuracy, sensitivity, and specificity can be calculated according to the discriminant formula using a newly prepared sample cohort for evaluation of the performance of the discriminant.

The present invention provides a polynucleotide for detection or diagnosis of a disease that is useful for diagnosis and treatment of uterine leiomyosarcoma, a method for detecting uterine leiomyosarcoma using the polynucleotide, and a kit and a device for detecting uterine leiomyosarcoma comprising the polynucleotide. According to conventional diagnosis, in particular, a subject without uterine leiomyosarcoma may be misdiagnosed as a uterine leiomyosarcoma patient, which leads to needless additional testing, or opportunities for treatment may be lost because of a failure to detect a uterine leiomyosarcoma patient. According to the present invention, in contrast, uterine leiomyosarcoma can be accurately identified in a non-invasive manner with a small amount of a sample, irrespective of the stage, the degree of infiltration, the histological grade, and the primary or recurrent phase. Specifically, the present invention provides a kit or device for diagnosis of a disease useful for diagnosis and treatment of uterine leiomyosarcoma with the use of highly accurate uterine leiomyosarcoma markers, and a method for determining (or detecting) uterine leiomyosarcoma.

A set of genes for diagnosis may comprise any combination selected from among, for example, one, two, or more of the above polynucleotides comprising a nucleotide sequence shown in any of SEQ ID NOs: 1 to 7 or a complementary sequence thereof. In addition, a discriminant formula is constructed using the expression levels of the set of genes for diagnosis in samples from patients diagnosed to have uterine leiomyosarcoma as a result of tissue diagnosis and samples from test subjects without uterine leiomyosarcoma. As a result, whether or not a subject, from which an unknown sample is provided, has uterine leiomyosarcoma can be determined by measuring expression levels of the set of genes for diagnosis in the unknown sample.

The kit and the method, etc. according to the present invention enable detection of uterine leiomyosarcoma with high sensitivity, which leads to early detection of uterine leiomyosarcoma. As a result, early treatment becomes possible, and viability can be improved to a significant extent. In addition, it becomes possible to avoid loss of treatment opportunities caused by a failure to detect uterine leiomyosarcoma or needless additional testing performed based on misdiagnosis of a subject without uterine leiomyosarcoma as a uterine leiomyosarcoma patient, which are necessitated due to high fluctuation observed among persons involved in urine cytology or different results attained because of subjective views of operators of prostate endoscopy.

EXAMPLES

The present invention is described in greater detail with reference to the following examples, although the technical scope of the present invention is not limited to these examples.

[Reference Example]

<Collection of samples>

The informed consents were obtained from 6 uterine leiomyosarcoma patients, 2 uterus adenosarcoma patients, 2 uterus endometrial stromal sarcoma patients (ESS), 1 patient with smooth muscle tumor of unknown malignancy (STUMP), and 18 uterine fibroid patients, who were diagnosed by postoperative tissue pathological inspection, and serum samples were respectively obtained therefrom.

The age distribution of these cases was as follows: uterine leiomyosarcoma patients: 59.7-year-old on average (± 16.5-year-old); uterus adenosarcoma patients: 46.0-year-old on average (± 5.7-year-old); uterus endometrial stromal sarcoma patients: 43.0-year-old on average (± 7.1-year-old); patients with smooth muscle tumor of unknown malignancy (STUMP): 65-year-old; and uterine fibroid patients: 46.8-year-old on average (± 11.1-year-old). Further, the concentration of lactic acid dehydrogenase (LDH) in each disease was measured. The clinical information is collectively shown in Table 2.

[Table 2]

| | Number of cases | Age (years) | LDH concentration |
|---|---|---|---|
| Uterine leiomyosarcoma | 6 | 59.7±16.5 | 219.0±83.2 |
| Uterus adenosarcoma | 2 | 46.0±5.7 | 186.5±65.8 |
| Uterus endometrial stromal sarcoma | 2 | 43.0±7.1 | 197.5±37.5 |

(continued)

| | Number of cases | Age (years) | LDH concentration |
|---|---|---|---|
| Leiomyosarcoma of unknown malignancy | 1 | 65 | 281 |
| Uterine fibroid | 18 | 47.4±10.7 | 286.0±46.3 |

<Extraction of Total RNA>

Total RNA was obtained using a reagent for RNA extraction in 3D-Gene (registered trademark) RNA extraction reagent from liquid sample kit (Toray Industries, Inc., Japan) according to the protocol provided by the manufacturer from 300 μl each of the serum samples obtained from the 29 subjects in total.

<Measurement of Gene Expression Level>

miRNA in the total RNA, which was obtained from each of the serum samples of the 29 subjects, was fluorescence-labeled with the use of 3D-Gene (registered trademark) miRNA Labeling kit (Toray Industries, Inc.) according to the protocol provided by the manufacturer. The oligo DNA chip used was 3D-Gene (registered trademark) Human miRNA Oligo chip (Toray Industries, Inc.) with attached probes having sequences complementary to 2,588 miRNAs among the miRNAs registered in miRBase Release 21. Hybridization was carried out under stringent conditions, followed by washing, in accordance with the protocol provided by the manufacturer. The DNA chip was scanned using 3D-Gene (registered trademark) scanner (Toray Industries, Inc.) to obtain images. Fluorescence intensity was digitized using 3D-Gene (registered trademark) Extraction (Toray Industries, Inc.).

The digitized fluorescence intensity was used to calculate the expression level of the genes detected in the manner described below. At the outset, the highest 5% and the lowest 5% of signal intensities at a plurality of negative control spots were excluded, the value; mean + 2 × standard error, thereof was calculated, and genes exhibiting signal intensities higher than the calculated value were regarded as being detected. From the signal intensity of the detected gene, the average signal intensity at the negative control spots from which the highest 5% and the lowest 5% of signal intensities had been excluded was subtracted, the calculated value was converted to a logarithmic value having a base of 2 and used as a gene expression level. For data normalization, the average of 3 types of miRNAs that are reported as endogenous controls: miR-149-3p, miR-2861, and miR-4463 (Shimomura et al., Cancer Science, 2016, Vol. 107, pp. 326-34), were used to normalize the data of different samples. The normalized data of the genes that were not detected above were converted to a logarithmic value 00.1 having a base of 2. Thus, signal intensities indicating the comprehensive gene expression levels of the miRNAs in the serum samples obtained from the 29 subjects described above were obtained.

A method of identifying uterine leiomyosarcoma was constructed step by step as described below. That is, the uterine leiomyosarcoma patients and the other patients were each divided into half, to prepare a search cohort and a validation cohort. The search cohort was subjected to Leave One Out Cross Validation, to select marker candidates, which were verified in the validation cohort.

Calculation and statistical analysis using the digitized gene expression levels of the miRNAs were carried out using R language 3.1.2 (R Foundation for Statistical Computing, https://www.R-project.org/.), compute.es package 0.2-4, hash package 2.2.6, MASS package 7.3.45, mutoss package 0.1-10, pROC package 1.8, and IBM SPSS Statistics version 22 (IBM Japan).

[Example 1]

<Analysis of identification of uterine leiomyosarcoma using discriminant formulae using 7 types of miRNA>

In this example, miRNAs were extracted as marker candidates in the training cohort, to perform clustering analysis using 7 types of miRNAs extracted.

In order to acquire more reliable diagnostic markers from among a total of 2,588 types of miRNAs existing in humans (analytes), specifically, all the samples were divided into the training cohort and the validation cohort, the differences in expression levels of miRNAs between the uterine leiomyosarcoma group and the other groups were compared in the training cohort using a cross validation method (Leave one out cross validation), and the number of candidate markers was first reduced in the training cohort. MiRNAs without a gene expression level of $2^6$ or larger in 50 % or more of the samples in either the positive sample group (uterine leiomyosarcoma patients) or the negative sample group (non-uterine leiomyosarcoma patients) were excluded because of low signal intensity and a lack of reliability, and remaining miRNAs were subjected to the subsequence procedure. The cross validation was then performed, and miRNAs with the accuracy of less than 0.75 and the difference in average between both groups of not exceeding 0.5 were eliminated. The 7 types of miRNAs remaining in the end were identified as marker candidates (Table 3).

Then, all the samples were subjected to clustering analysis using these 7 types of miRNAs. As a result, a cluster including a uterine leiomyosarcoma patient group was formed almost specifically (Fig. 2).

[Example 2]

Next, Fisher's discriminant analysis was performed on the measured expression levels of the 7 types of miRNAs in the training cohort obtained in the method according to Example 1, and cross validation was also performed to construct discriminant formulae to determine the presence or absence of uterine leiomyosarcoma.

As a result, for example, AUC of a polynucleotide consisting of the nucleotide sequence shown in SEQ ID NO: 1 was 0.61 in the training cohort, and the CV (Cross Validation) score was 0.75 (Table 3). Likewise, the AUCs of polynucleotides consisting of the nucleotide sequences shown in SEQ ID NOs: 2 to 7 were respectively 0.71, 0.71, 0.71, 0.63, 0.64, and 0.73 in the training cohort (Table 3).

[Table 3]

| SEQ ID NO: | micro RNA | Coefficient in discriminant formula | Constant in discriminant formula | AUC | CV score | Fold change ($log_2$) |
|---|---|---|---|---|---|---|
| 1 | hsa-miR-1246 | -0.73 | 6.42 | 0.61 | 0.75 | -0.91 |
| 2 | hsa-miR-4430 | -1.39 | 8.87 | 0.71 | 0.82 | -0.64 |
| 3 | hsa-miR-4485-5p | -0.69 | 3.87 | 0.71 | 0.75 | -1.08 |
| 4 | hsa-miR-451a | -0.38 | 1.03 | 0.71 | 0.75 | -2.88 |
| 5 | hsa-miR-4635 | -0.63 | 3.34 | 0.63 | 0.75 | -1.26 |
| 6 | hsa-miR-6511b | -0.77 | 3.86 | 0.64 | 0.79 | -1.15 |
| 7 | hsa-miR-191-5p | -0.45 | 2.77 | 0.73 | 0.75 | -2.52 |

In addition, a discriminant formula was identified by designating a specific discriminant threshold, and the markers were evaluated in terms of sensitivity and specificity. When a value obtained by the discriminant formula prepared with the use of the expression level of a polynucleotide consisting of the nucleotide sequence shown in SEQ ID NO: 1 (-0.73 × [hsa-miR-1246] + 6.42, in the second row of Table 3) is a positive value, for example, the subject can be evaluated to be with uterine leiomyosarcoma. In the case of a negative value, the subject can be evaluated to be without uterine leiomyosarcoma.

[Example 3]

<Analysis of identification of uterine leiomyosarcoma using discriminant formulae using two types of miRNA>

In this example, the 7 types of miRNAs extracted as candidate markers in the search cohort were used in multiple combinations to prepare discriminant formulae in the training cohort, and performance of the discriminant formulae prepared in the validation cohort was examined. Specifically, the expression levels of the 7 types of miRNAs obtained in the training cohort were each subjected to the Fisher's linear discriminant analysis, and cross validation was also performed to construct discriminant formulae to determine the presence or absence of uterine leiomyosarcoma.

As a result, the discriminant capability of the discriminant formula using 7 types of polynucleotides consisting of the nucleotide sequences shown in SEQ ID NOs: 1 to 7 in combination examined in the training cohort was higher than the discriminant capability of a polynucleotide consisting of a single nucleotide sequence shown in Example 1 in the validation cohort.

As described in Example 1, for example, AUC of a polynucleotide consisting of the nucleotide sequence shown in SEQ ID No: 1 (hsa-miR-1246) was 0.61 (in the second row of Table 3). In contrast, AUC of a discriminant formula prepared with the use of polynucleotides consisting of the nucleotide sequences shown in SEQ ID NOs: 1 and 6 in combination (respectively, hsa-miR-1246 and hsa-miR-6511b) was 0.65 (in the fifth row of Table 4 below). Table 4 shows these results collectively.

[Table 4]

| Discriminant formula | AUC | CV score |
|---|---|---|
| (-0.98) × miR-4430 + (-0.30) × miR-4485-5p + 8.15 | 0.77 | 0.79 |
| (-0.62) × miR-4430 + (-0.28) × miR-451a + 5.34 | 0.76 | 0.79 |
| (-1.09) × miR-4430 + (-0.27) × miR-1246 | 0.71 | 0.79 |
| (-0.67) × miR-6511b-5p + (-0.14) × miR-1246 + 5.22 | 0.65 | 0.79 |
| (0.82) × miR-1246 + (-0.84) × miR-191-5p - 2.59 | 0.83 | 0.75 |
| (-0.50) × miR-4430 + (-0.35) × miR-191-5p + 5.45 | 0.76 | 0.75 |
| (-0.16) × miR-451 a + (-0.29) × miR-191-5p + 1.93 | 0.75 | 0.75 |
| (-0.92) × miR-4430 + (-0.33) × miR-4635+8.00 | 0.74 | 0.75 |
| (-0.31) × miR-451a + (-0.22) × miR-4485-5p + 2.98 | 0.74 | 0.75 |
| (-0.80) × miR-4430 + (-0.45) × miR-6511 b-5p + 7.89 | 0.72 | 0.75 |
| (-0.13) × miR-6511 b-5p + (-0.40) × miR-191-5p + 3.16 | 0.72 | 0.75 |
| (-0.05) × miR-4635 + (-0.43) × miR-191-5p + 2.89 | 0.72 | 0.75 |
| (-0.38) × miR-451 a + (0.01) × miR-4635 + 0.97 | 0.71 | 0.75 |
| (-0.23) × miR-6511b-5p + (-0.30) × miR-451 a + 2.77 | 0.7 | 0.75 |
| (-0.60) × miR-6511b-5p + (-0.20) × miR-4485-5p + 4.35 | 0.64 | 0.75 |
| (-0.32) × miR-4485-5p + (-0.40) × miR-4635 + 3.76 | 0.64 | 0.75 |
| (-0.51) × miR-4635 + (-0.19) × miR-1246 | 0.63 | 0.75 |
| (-0.66) × miR-6511b-5p + (-0.10) × miR-4635 +3.86 | 0.62 | 0.75 |
| (-0.47) × miR-4485-5p + (-0.38) × miR-1246 + 6.02 | 0.72 | 0.71 |

Further, AUC of a discriminant formula prepared with the use of all the 7 types of polynucleotides consisting of the nucleotide sequences shown in SEQ ID Nos: 1 to 7 in combination for uterine leiomyosarcoma (6 cases) and non-uterine leiomyosarcoma (a total of 23 cases including uterus adenosarcoma, uterus endometrial stromal sarcoma (ESS), smooth muscle tumor of unknown malignancy (STUMP), and uterine fibroid) was 0.97. At this time, a similar AUC of LDH was 0.60. As described above, uterine leiomyosarcoma can be identified with discrimination capability superior to that of LDH by the use of 7 types of polynucleotides in combination.

[Example 4]

<Identification of uterine leiomyosarcoma based on pathological classification by discriminant formula using miRNA>

In this example, discriminant formulae of miRNAs prepared and examined in Examples 1 and 2 were used to evaluate the discrimination capability by focusing on pathological features of uterine leiomyosarcoma. Specifically, discrimination of uterine leiomyosarcoma from uterus adenosarcoma, uterus endometrial stromal sarcoma (ESS), smooth muscle tumor of unknown malignancy (STUMP), and uterine fibroids was studied.

As a result, AUC of a discriminant formula prepared with the combination use of polynucleotides consisting of the nucleotide sequences shown in SEQ ID NOs: 1 and 7 in combination was 0.97, as shown in Fig. 3(A), which significantly exceeded AUC of LDH, 0.64. Further, this formula could classify and discriminate uterine leiomyosarcoma from uterus adenosarcoma, uterus endometrial stromal sarcoma (ESS), smooth muscle tumor of unknown malignancy (STUMP), and uterine fibroids, as shown in Fig. 3(B).

(Sensitivity of uterine leiomyosarcoma: 100%, specificity of ESS and uterus adenosarcoma 50%, specificity of STUMP: 100%, and specificity of uterine fibroids: 79%)

[Comparative Example 1]

This comparative example describes the miRNAs that were not extracted as marker candidates in the training cohort in the process of extracting 7 types of miRNAs in Example 1. In order to acquire more reliable diagnostic markers from among a total of 2,588 types of miRNAs existing in humans (analytes), specifically, all the samples were divided into the training cohort and the validation cohort, the differences in expression levels of miRNAs between the uterine leiomyosarcoma group and the other groups were compared in the training cohort using a cross validation method (Leave one out cross validation), and the number of candidate markers was first reduced in the training cohort. MiRNAs without a gene expression level of $2^6$ or larger in 50 % or more of the samples in either the positive sample group (uterine leiomyosarcoma patients) or the negative sample group (non-uterine leiomyosarcoma patients) were excluded because of low signal intensity and a lack of reliability, and remaining miRNAs were subjected to the subsequence procedure. The cross validation was then performed, and miRNAs with the accuracy of less than 0.75 and the difference in average between both groups of not exceeding 0.5 were eliminated.

In this process, the 9 types of micro RNAs shown in Fig. 5 of Non Patent Literature 1, that is, any of miR-130b-3p, miR-141-3p, miR-148a-3p, miR-152-3p, miR-202-3p, miR-205-5p, miR-210-3p, miR-31-5p, and miR-328-3p, in which variation in expression was 4 times or more in the samples of the uterine leiomyosarcoma patients as compared with the endometrium-derived cells as a control was not selected as a marker candidate in the training cohort.

All the publications, patents, and patent applications cited herein are incorporated herein by reference.

## Claims

1. A kit for detection of uterine leiomyosarcoma, comprising a nucleic acid capable of specifically binding to at least one polynucleotide selected from the group consisting of the following uterine leiomyosarcoma markers; miR-1246, miR-4430, miR-4485-5p, miR-451a, miR-4635, miR-6511b-5p, and miR-191-5p or a complementary strand of the polynucleotide.

2. The kit according to claim 1, wherein the nucleic acid is a polynucleotide selected from the group consisting of the following polynucleotides (a) to (e):

(a) a polynucleotide consisting of a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(b) a polynucleotide comprising a nucleotide sequence shown in any of SEQ ID NOs: 1 to 7;
(c) a polynucleotide consisting of a nucleotide sequence complementary to a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(d) a polynucleotide comprising a nucleotide sequence complementary to a nucleotide sequence shown in any of SEQ ID NOs: 1 to 7 or a nucleotide sequence derived therefrom by the replacement of u with t; and
(e) a polynucleotide hybridizing under stringent conditions to any of the polynucleotides (a) to (d).

3. A device for detection of uterine leiomyosarcoma, comprising a nucleic acid capable of specifically binding to at least one polynucleotide selected from the group consisting of the following uterine leiomyosarcoma markers; miR-1246, miR-4430, miR-4485-5p, miR-451a, miR-4635, miR-6511b-5p, and miR-191-5p or a complementary strand of the polynucleotide.

4. The device according to claim 3, wherein the nucleic acid is a polynucleotide selected from the group consisting of the following polynucleotides (a) to (e):

(a) a polynucleotide consisting of a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(b) a polynucleotide comprising a nucleotide sequence shown in any of SEQ ID NOs: 1 to 7;
(c) a polynucleotide consisting of a nucleotide sequence complementary to a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(d) a polynucleotide comprising a nucleotide sequence complementary to a nucleotide sequence shown in any of SEQ ID NOs: 1 to 7 or a nucleotide sequence derived therefrom by the replacement of u with t; and
(e) a polynucleotide hybridizing under stringent conditions to any of the polynucleotides (a) to (d).

5. The device according to claim 3 or 4, which is used for measurement by a hybridization technique.

6. The device according to claim 5, wherein the hybridization technique is a nucleic acid array technique.

7. A method for detecting uterine leiomyosarcoma, comprising: measuring an expression level of at least one polynucleotide selected from the group consisting of the following uterine leiomyosarcoma markers; miR-1246, miR-4430, miR-4485-5p, miR-451a, miR-4635, miR-6511b-5p, and miR-191-5p, in a sample from a subject; and evaluating *in vitro* whether or not the subject has uterine leiomyosarcoma using the measured expression level.

8. The method according to claim 7 comprising: plugging the gene expression level of the one or more polynucleotide in the sample from the subject into a discriminant formula capable of discriminating the presence or absence of uterine leiomyosarcoma distinctively, wherein the discriminant formula is created using gene expression levels in samples from subjects known to have uterine leiomyosarcoma and gene expression levels in samples from subjects having no uterine leiomyosarcoma as training samples; and thereby evaluating as to the presence or absence of uterine leiomyosarcoma *in vitro.*

9. The method according to claim 7 or 8, comprising: measuring an expression level of the polynucleotide using a nucleic acid capable of specifically binding to the polynucleotide or a complementary strand of the polynucleotide, wherein the nucleic acid is a polynucleotide selected from the group consisting of the following polynucleotides (a) to (e):

(a) a polynucleotide consisting of a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(b) a polynucleotide comprising a nucleotide sequence shown in any of SEQ ID NOs: 1 to 7;
(c) a polynucleotide consisting of a nucleotide sequence complementary to a nucleotide sequence shown in any of SEQ ID NOs: 1 to 7 or a nucleotide sequence derived therefrom by the replacement of u with t, a variant thereof, a derivative thereof, or a fragment thereof comprising 15 or more consecutive nucleotides;
(d) a polynucleotide comprising a nucleotide sequence complementary to a nucleotide sequence shown in any of SEQ ID NOs: 1 to 7 or a nucleotide sequence derived therefrom by the replacement of u with t; and
(e) a polynucleotide hybridizing under stringent conditions to any of the polynucleotides (a) to (d).

10. The method according to any one of claims 7 to 9, comprising: measuring an expression level of a target gene in the sample from the subject using the kit according to claim 1 or 2 or the device according to any one of claims 3 to 6, wherein the kit or the device comprises a nucleic acid capable of specifically binding to the polynucleotide or a complementary strand of the polynucleotide.

11. The method according to any of claims 7 to 10, wherein the subject is a human.

12. The method according to any of claims 7 to 11, wherein the sample is blood, serum, or plasma.

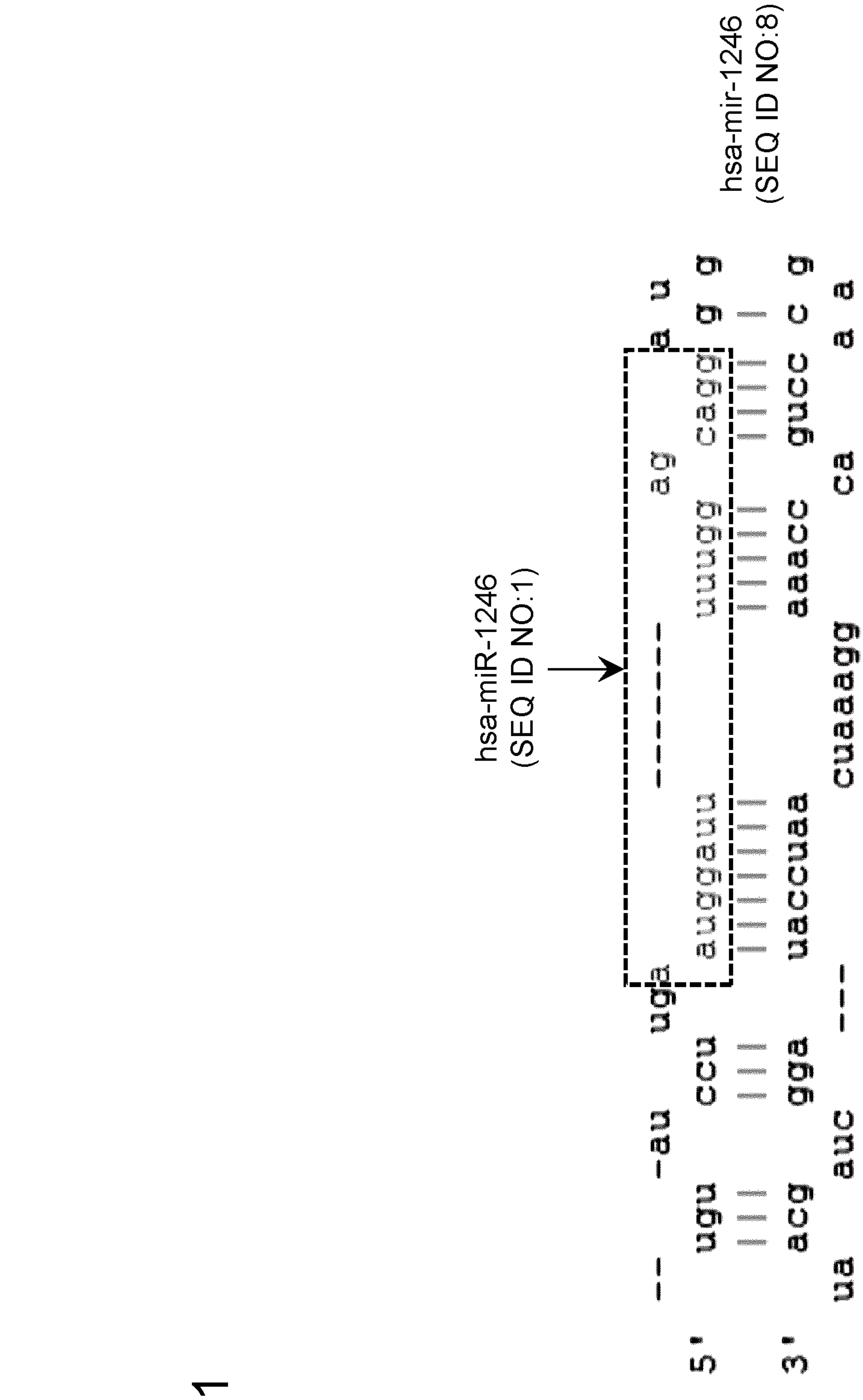
hsa-mir-1246
(SEQ ID NO:8)
hsa-miR-1246
(SEQ ID NO:1)
-- -au uga ------- ag a u
5' ugu ccu auggauu uuugg cagg g g
3' acg gga uaccuaa aaacc gucc c g
ua auc --- cuaaagg ca a a

Fig. 1

Fig. 2

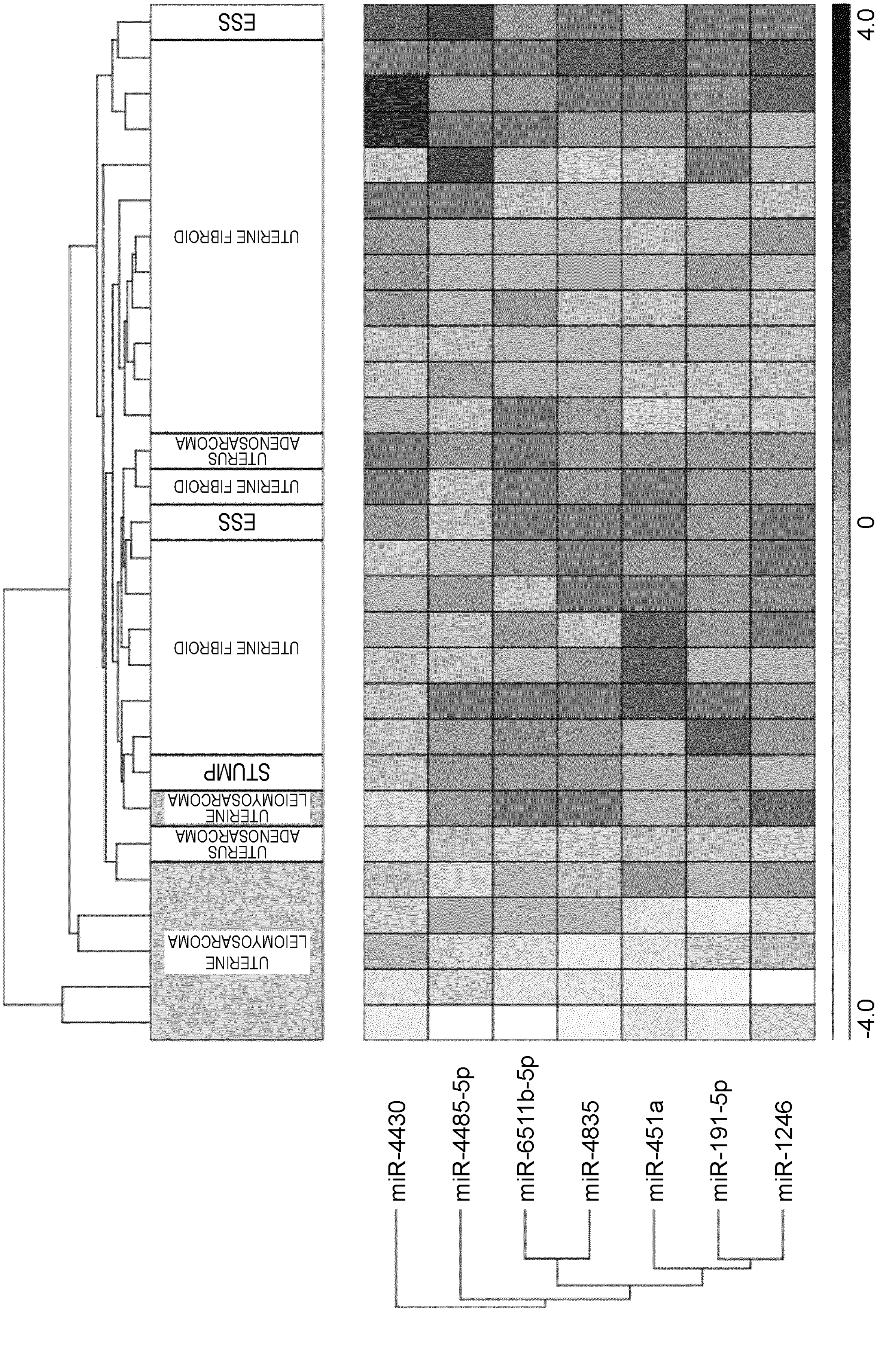
ESS
UTERINE FIBROID
UTERUS
ADENOSARCOMA
UTERINE FIBROID
ESS
UTERINE FIBROID
STUMP
UTERINE
LEIOMYOSARCOMA
UTERUS
ADENOSARCOMA
UTERINE
LEIOMYOSARCOMA
4.0
0
-4.0
miR-4430
miR-4485-5p
miR-6511b-5p
miR-4835
miR-451a
miR-191-5p
miR-1246

Fig. 3

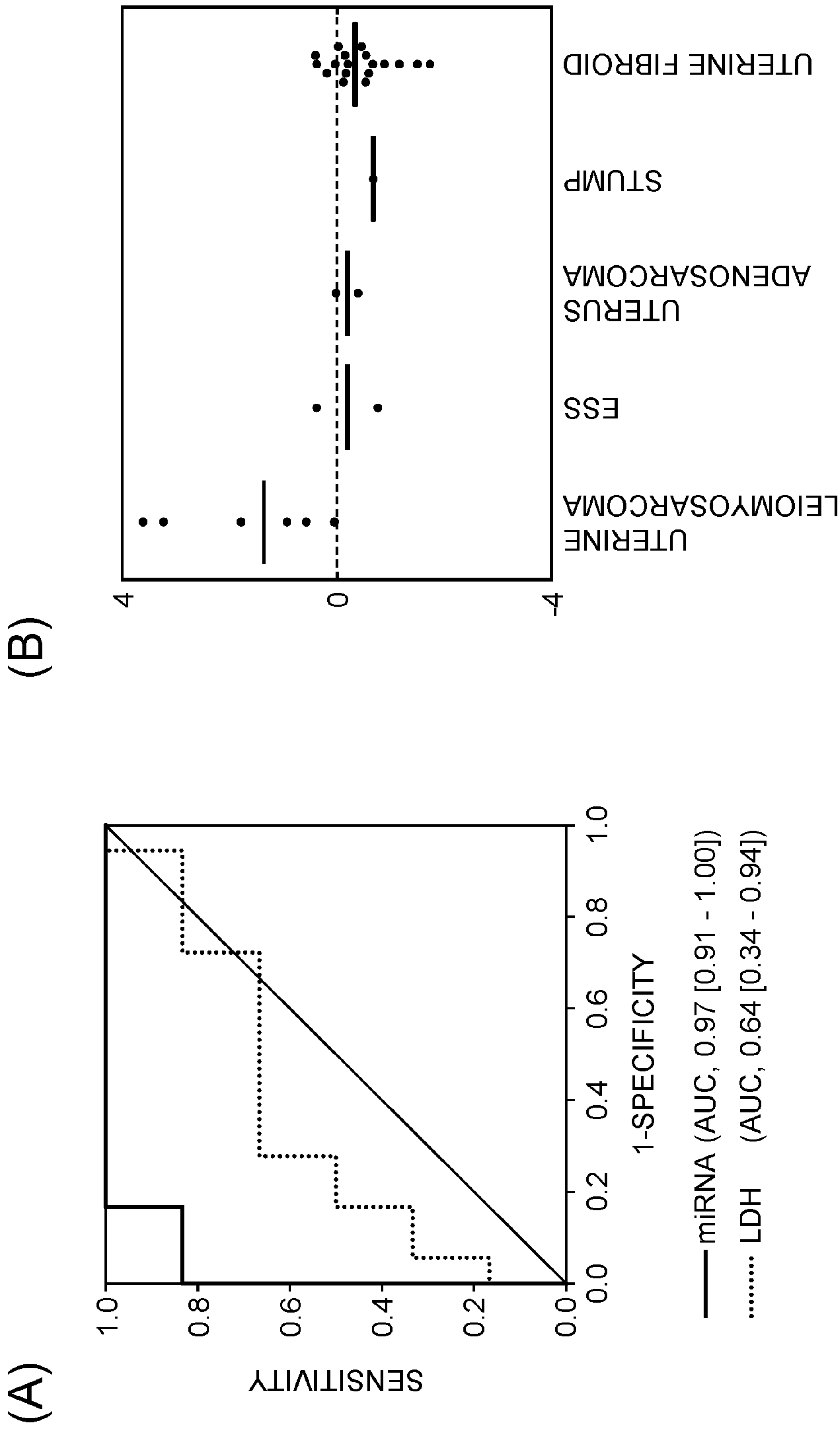
(A)
SENSITIVITY
1.0
0.8
0.6
0.4
0.2
0.0
0.0
0.2
0.4
0.6
0.8
1.0
1-SPECIFICITY
miRNA (AUC, 0.97 [0.91 - 1.00])
LDH (AUC, 0.64 [0.34 - 0.94])
(B)
4
0
-4
UTERINE LEIOMYOSARCOMA
ESS
UTERUS ADENOSARCOMA
STUMP
UTERINE FIBROID

## INTERNATIONAL SEARCH REPORT

International application No.
PCT/JP2020/009566

A. CLASSIFICATION OF SUBJECT MATTER
C12N 15/09(2006.01)i; C12Q 1/6813(2018.01)i; C12Q 1/6886(2018.01)i
FI: C12Q1/6813; C12N15/09 200; C12Q1/6886 Z ZNA

According to International Patent Classification (IPC) or to both national classification and IPC

B. FIELDS SEARCHED

Minimum documentation searched (classification system followed by classification symbols)
C12N15/09; C12Q1/6813; C12Q1/6886

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN); PubMed

C. DOCUMENTS CONSIDERED TO BE RELEVANT

| Category* | Citation of document, with indication, where appropriate, of the relevant passages | Relevant to claim No. |
|---|---|---|
| X | YOKOI, A. et al., "Integrated extracellular microRNA profiling for ovarian cancer screening", NATURE COMMUNICATIONS, 2018, vol. 9, 4319 (10 pages, Suppl. Info.), abstract, discussion, lines 1-6 abstract, discussion, lines 1-6 | 3-6<br>1-11 |
| Y | abstract, discussion, lines 1-6 | 12 |
| A | abstract, discussion, lines 1-6 | |
| X | ONO, S. et al., "Circulating microRNA Biomarkers as Liquid Biopsy for Cancer Patients: Pros and Cons of Current Assays", Journal of Clinical Medicine, 2015, vol. 4, pp. 1890-1907, abstract, section "table 3, 4. 2" abstract, table 3, section "4.2" | 3-6 |
| Y | abstract, table 3, section "4.2" | 1-11 |
| A | abstract, table 3, section "4.2" | 12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 May 2020 (26.05.2020) | 09 June 2020 (09.06.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/009566

C (Continuation). DOCUMENTS CONSIDERED TO BE RELEVANT

| Category* | Citation of document, with indication, where appropriate, of the relevant passages | Relevant to claim No. |
|---|---|---|
| Y | RAVID, Y. et al., "Uterine leiomyosarcoma and endometrial stromal sarcoma have unique miRNA signatures", Gynecologic Oncology, 2016, vol. 140, pp. 512-517, abstract, table 2, etc. abstract, table 2, etc. | 1-11 |
| A | abstract, table 2, etc. | 12 |
| Y | ALMEIDA, B. C. et al., "Oncomirs Expression Profiling in Uterine Leiomyosarcoma cells", International Journal of Molecular Sciences, 2018, vol. 19, 52 (13 pages), abstract, fig. 6, etc. abstract, fig. 6, etc. | 1-11 |
| A | abstract, fig. 6, etc. | 12 |
| Y | KOWALEWSKA, M. et al., "microRNAs in uterine sarcomas and mixed epithelial-mesenchymal uterine tumors: a preliminary report", Tumor Biology, 2013, vol. 34, pp. 2153-5160, abstract, table 3, etc. abstract, table 3, etc. | 1-11 |
| A | abstract, table 3, etc. | 12 |
| P,X | YOKOI, A. et al., "Serum microRNA profile enables preoperative diagnosis of uterine leiomyosarcoma", Cancer Science, 10 October 2019, vol. 110, pp. 3718-3726, abstract, table 2, etc. | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018044979 A
- JP 2019041982 A


### Non-patent literature cited in the description


- **DE ALMEIDA B. et al.** *Int. J. Mol. Sci.,* 2018, vol. 19 (1), 52
- **ZHENG ZHANG et al.** *J. Comput. Biol.,* 2000, vol. 7, 203-214
- **ALTSCHUL, S.F. et al.** *Journal of Molecular Biology,* 1990, vol. 215, 403-410
- **PEARSON, W.R. et al.** *Proc. Natl. Acad. Sci. U.S.A.,* 1988, vol. 85, 2444-2448
- **NIELSEN, P.E. et al.** *Science,* 1991, vol. 254, 1497-500
- **OBIKA, S et al.** *Tetrahedron Lett.,* 1998, vol. 39, 5401-5404
- **MORIN RD et al.** *GenomeRes,* 2008, vol. 18, 610-621
- **JIMA DD et al.** *Blood,* 2010, vol. 116, e118-e127
- **ALTUVIA Y et al.** *Nucleic Acids Res.,* 2005, vol. 33, 2697-2706
- **PERSSON H et al.** *Cancer Res,* 2011, vol. 71, 78-86
- **LI Y et al.** *Gene,* 2012, vol. 497, 330-335
- **LAGOS-QUINTANA M et al.** *RNA,* 2003, vol. 9, 175-179
- **MORIN RD et al.** *Genome Res.,* 2008, vol. 18, 610-621
- **AUSUBEL et al.** Current Protocols in Molecular Biology. John Willey & Sons, 1993
- **SAMBROOK et al.** Molecular Cloning A Laboratory Manual. Cold Spring Harbor Laboratory Press, 1989
- **SAMBROOK, J. ; RUSSEL, D.** Molecular Cloning, A LABORATORY MANUAL. Cold Spring Harbor Laboratory Press, 15 January 2001, vol. 1, 7.42-7.45
- **SAMBROOK, J. , RUSSEL, D.** Molecular Cloning, A LABORATORY MANUAL. Cold Spring Harbor Laboratory Press, 15 January 2001, vol. 2, 8.9-8.17
- **RICK KAMPS et al.** *Int. J. Mol. Sci.,* 2017, vol. 18 (2), 308
- *Int. Neurourol. J.,* 2016, vol. 20 (2), S76-83
- **SPEED T.** Statistical analysis of gene expression microarray data. Chapman and Hall/CRC
- **CAUSTON H. C. et al.** A beginner's guide: Microarray gene expression data analysis. Blackwell publishing
- **BOLSTAD, B.M. et al.** *Bioinformatics,* 2003, vol. 19, 185-193
- **VENABLES, W. N. et al.** Modern Applied Statistics with S. Springer, 2002
- **TAKAFUMI KANAMORI et al.** *Pattern Recognition,* 2009
- **RICHARD O. et al.** Pattern Classification. Wiley-Interscience, 2000
- **V. VAPNIK.** The Nature of Statistical Leaning Theory. Springer, 1995
- Statistics of pattern recognition and learning - New concepts and approaches. **HIDEKI ASO et al.** Frontier of Statistical Science. Iwanami Shoten, Publishers, 2004, vol. 6
- **NELLO CRISTIANINI et al.** *Introduction to SVM,* 2008
- **C. CORTES et al.** *Machine Learning,* 1995, vol. 20, 273-297
- **TIBSHIRANI R.** *J. R. Stat. Soc. Ser. B,* 1996, vol. 58, 267-88
- **YASUSHI NAGATA et al.** Basics of statistical multiple comparison methods. Scientist Press Co., Ltd, 2007
- **FUREY TS et al.** *Bioinformatics.,* 2000, vol. 16, 906-14
- **SHIMOMURA et al.** *Cancer Science,* 2016, vol. 107, 326-34